# EUROPÄISCHE PATENTSCHRIFT

(11) **EP 3 586 721 B1**
(45) Veröffentlichungstag und Bekanntmachung des Hinweises auf die Patenterteilung: **08.07.2020**
(21) Anmeldenummer: 19182842.5
(22) Anmeldetag: 27.06.2019
(51) Int. Cl.: A61B 3/02, A61B 3/00, G02C 7/02, G06N 20/00, G02C 11/00, G06N 3/04, G06N 3/08

(54) **VERFAHREN ZUM OPTIMIEREN EINES OPTISCHEN HILFSMITTELS MITTELS AUTOMATISCHER BESTIMMUNG SUBJEKTIVER SEHLEISTUNG**
METHOD FOR OPTIMISING AN OPTICAL AID USING AUTOMATIC DETERMINATION OF SUBJECTIVE VISION
PROCÉDÉ D'OPTIMISATION D'UN MOYEN OPTIQUE À L'AIDE DE LA DÉTERMINATION AUTOMATIQUE DES CAPACITÉS VISUELLES SUBJECTIVES

(30) Priorität: 29.06.2018 EP 18180876
(43) Veröffentlichungstag der Anmeldung: 01.01.2020
(73) Patentinhaber: Carl Zeiss Vision International GmbH, 73430 Aalen (DE)
(72) Erfinder: LEUBE, Alexander, 72072 Tübingen, (DE); LEIBIG, Christian, 81543 München, (DE); OHLENDORF, Arne, 72072 Tübingen, (DE); WAHL, Siegfried, 73072 Donzdorf, (DE)
(74) Vertreter: Louis Pöhlau Lohrentz

(56) Entgegenhaltungen:
- WO-A1-2018/022521
- US-A1- 2017 273 552
- US-A1- 2017 371 178
- US-B1- 8 690 325

## Beschreibung

Die Erfindung betrifft ein Verfahren zum Optimieren eines optischen Hilfsmittels mittels automatischer Bestimmung subjektiver Sehleistung.

Bekannte Verfahren zur Bestimmung subjektiver Sehleistung, beispielsweise des Auflösungsvermögens eines Auges einer Person, beruhen auf der Annahme, dass es einen Zusammenhang zwischen optischen Fehlern und der zu erwartenden Sehleistung gibt. Die bekannten Verfahren modellieren das visuelle System mittels verschiedener optischer und neuronaler Filterfunktionen. Diese Modellierung basiert auf subjektiven Probandenmessungen. Subjektive Probandenmessungen sind langwierig und unterliegen einer Verfälschung der Ergebnisse durch störende Einflüsse, beispielsweise durch eine Ermüdung des Probanden. Weiterhin besitzen die Filterfunktionen einstellbare Gewichtungen, die sinnvoll anzupassen sind. Insbesondere kann die neuronale Transferfunktion hochgradig nichtlinear und damit schwer zu beschreiben sein. Neuronale Transferfunktionen können individuell stark verschieden sein. Bei den bekannten Verfahren ist es daher nicht gewährleistet, dass hinreichend genaue Ergebnisse erzielt werden. Entsprechend ist auch das Optimieren eines optischen Hilfsmittels mit bekannten Verfahren zur Bestimmung subjektiver Sehleistung langwierig, anfällig für störende Einflüsse und nicht ausreichend genau.

Der vorliegenden Erfindung liegt die Aufgabe zugrunde, die Nachteile aus dem Stand der Technik zu beseitigen. Insbesondere soll ein Verfahren zum Optimieren eines optischen Hilfsmittels bereitgestellt werden, bei dem die subjektive Sehleistung schnell, ohne störende Einflüsse und mit einer guten Genauigkeit bestimmt wird. Weiterhin sind ein Herstellungsverfahren für ein entsprechend optimiertes optisches Hilfsmittel sowie eine Vorrichtung zum Herstellen eines entsprechend optimierten optischen Hilfsmittels anzugeben.

Erfindungsgemäß wird diese Aufgabe in Bezug auf das Optimierungsverfahren mit dem Gegenstand des Anspruchs 1, in Bezug auf das Herstellungsverfahren mit dem Gegenstand des Anspruchs 10 und in Bezug auf die Vorrichtung mit dem Gegenstand des Anspruchs 12 gelöst. Vorteilhafte Ausgestaltungen der Erfindung sind in den abhängigen Ansprüchen angegeben.

Das erfindungsgemäße Verfahren zum Optimieren eines optischen Hilfsmittels führt eine automatische Bestimmung subjektiver Sehleistung unter Verwendung von maschinellem Lernen durch und umfasst die folgenden Schritte:
a) Bereitstellen zumindest eines Trainingsdatensatzes,
b) Trainieren eines künstlichen neuronalen Netzwerks mittels des zumindest einen Trainingsdatensatzes,
c) Generisches Erstellen eines Stimulusbilds,
d) Parametrisieren des Stimulusbilds, wobei eine erste und eine zweite Parametrisierung vorgenommen wird, und wobei vorzugsweise die erste Parametrisierung des Stimulusbilds zumindest durch eine Bildgröße des Stimulusbilds, einen Stimuluskontrast und/oder eine Stimulusform erfolgt,
e) Bereitstellen einer Erkennungswahrscheinlichkeitsfunktion,
f) Klassifizieren des Stimulusbilds durch das trainierte neuronale Netzwerk zum Bereitstellen einer Klassifizierung des Stimulusbilds,
g) Bestimmen einer Erkennungswahrscheinlichkeit für die im Schritt d) vorgenommene erste Parametrisierung auf der Grundlage der Klassifizierung des Stimulusbilds,
h) Anpassen der ersten Parametrisierung des Stimulusbilds auf der Grundlage der bestimmten Erkennungswahrscheinlichkeit,
i) Klassifizieren des angepassten Stimulusbilds durch das trainierte neuronale Netzwerk zum Bereitstellen einer Klassifizierung des angepassten Stimulusbilds,
j) Bestimmen der Erkennungswahrscheinlichkeit für die im Schritt h) angepasste erste Parametrisierung auf der Grundlage der Klassifizierung des angepassten Stimulusbilds,
k) Wiederholen der Schritte h) bis j) bis zum Erreichen eines Abbruchkriteriums in Bezug auf ein Auffinden einer Schwelle der subjektiven Sehleistung,
l) Bestimmen der Schwelle der subjektiven Sehleistung,
m) mehrfach wiederholtes Durchlaufen der Schritte d) bis I) mit jeweils unterschiedlicher zweiter Parametrisierung, wobei die zweite Parametrisierung des Stimulusbilds jeweils das Anwenden zumindest einer Eigenschaft des optischen Hilfsmittels auf das Stimulusbild umfasst,
n) Optimieren des optischen Hilfsmittels durch Auswählen der zweiten Parametrisierung mit der beim Schritt I) höchsten bestimmten subjektiven Sehleistung.

Bei dem Verfahren handelt es sich um ein in-vitro-Verfahren; das heißt keiner der Verfahrensschritte wird am oder im menschlichen Körper durchgeführt.

Die Verfahrensschritte werden vorzugsweise in der vorgegebenen Reihenfolge a) - n) durchgeführt.

Der Begriff "optisches Hilfsmittel" wird in der vorliegenden Patentanmeldung als Sammelbegriff verwendet. Der Begriff "optisches Hilfsmittel" umfasst Brillengläser, Brillenglasrohlinge, Gleitsichtgläser, Kontaktlinsen, intraokulare Linsen, Objektive, Fernglasoptiken sowie Kombinationen verschiedener Optiken. Dabei werden unter Brillengläsern auch Brillenglasrohlinge und Gleitsichtgläser verstanden. Vorzugsweise ist das "optische Hilfsmittel" ein Brillenglas oder eine Brille mit zwei Brillengläsern.

Der Begriff "optisches System" wird in der vorliegenden Patentanmeldung in der Bedeutung optisches Hilfsmittel und/oder menschliches Auge verstanden.

Das genannte menschliche Auge ist in der vorliegenden Patentanmeldung das Auge einer bestimmten Person, für das spezifisch das erfindungsgemäße Optimieren eines optischen Hilfsmittels (insbesondere eines Brillenglases) durchgeführt wird.

Der Begriff "Sehleistung" wird in der vorliegenden Patentanmeldung austauschbar mit dem Begriff "Performanz" bzw. "visuelle Performanz" verwendet. Die "Sehleistung" bzw. die "(visuelle) Performanz" steht für die Fähigkeit eines optischen und/oder neuronalen Systems, das heißt für die Fähigkeit des Auges (zusammen mit dem Sehnerv und dem Gehirn) und/oder eines optischen Hilfsmittels, ein gegebenes Stimulusbild hinreichend zu verarbeiten und/oder aufzulösen. Die "Sehleistung" bzw. die "(visuelle) Performanz" ist dabei abhängig von der Art der Parametrisierung des Stimulusbilds. Die "Sehleistung" bzw. die "(visuelle) Performanz" bezieht sich hinsichtlich des Parameters Bildgröße auf das Auflösungsvermögen, hinsichtlich des Parameters Kontrast auf die Fähigkeit hinreichend niedrige oder hohe Abstufungen von Kontrasten zu verarbeiten und hinsichtlich des Parameters Form auf die Fähigkeit Formen unterscheiden zu können. Der Begriff "subjektive Sehleistung" bezieht sich dabei auf die Sehleistung einer Person. Im Fall des Parameters Bildgröße ist eine äquivalente Definition des Parameters durch den zur Bildgröße korrespondierenden Sehwinkel möglich. Darüber hinaus kann sich der Parameter Bildgröße bzw. Sehwinkel auf das gesamte Stimulusbild beziehen oder lediglich auf die Erstreckung eines kritischen Details innerhalb des Stimulusbilds. Ein kritisches Detail ist beispielsweise die Breite der Lücke bei einem Landoltring.

Das Stimulusbild ist vorzugsweise ein Landolt-C (auch Landoltring genannt). Dabei handelt es sich um ein rundes C, dessen Lücke verschiedene Ausrichtungen aufweisen kann, insbesondere die acht verschiedenen Ausrichtungen "0°", "45°", "90°", "135°", "180°", "225°", "270°" oder "315°". Das Stimulusbild kann alternativ dazu beispielsweise ein Snellen-Haken (auch E-Haken genannt), ein Buchstabe, eine Zahl, ein Kontrastmuster oder ein natürliches Bild sein. Unter einem natürlichen Bild wird insbesondere ein Bild mit spezifischen einer natürlichen Umgebung nachempfunden Verteilungen von auftretenden Sehwinkeln verstanden. Verallgemeinert gesprochen ist jedes zur Sehleistungbestimmung geeignete Stimulusbild zur Durchführung der Erfindung geeignet. Vorzugsweise sind die Größenverhältnisse bei den genannten alternativen Stimulusbildern den Größenverhältnissen bei einem Landoltring nachempfunden. Das Stimulusbild kann synthetisch erstellt werden.

Jedes Stimulusbild trägt zumindest ein zu erkennendes Feature. Beim Landoltring ist das Feature beispielsweise die Ausrichtung der Lücke. Bei einer Zahl ist das Feature, um welche Zahl es sich handelt.

Unter einer Parametrisierung wird im Sinn dieser Anmeldung der konkrete Wert bzw. die konkreten Werte des bzw. der betreffenden Parameter verstanden. Allgemeiner gesprochen ist die Parametrisierung ein konkreter Punkt in einem ein- oder mehrdimensionalen Parameterraum. Handelt es sich bei dem betrachteten Parameter beispielsweise um die Bildgröße, so ist die Parametrisierung ein konkreter Wert der Bildgröße. Der Bildgröße entspricht ein Sehwinkel, unter dem das Stimulusbild bei standardisierten Bedingungen (insbesondere bei festem Abstand) einer Person (bzw. einem Probanden) präsentiert würde.

Der im Schritt a) bereitgestellte Trainingsdatensatz spiegelt vorzugsweise die subjektive Sehleistung einer Vielzahl von realen Probanden beim Erkennen von Stimulusbildern wider. Zum Erstellen des Trainingsdatensatzes wird einem ersten realen Probanden beispielsweise ein Stimulusbild auf einem Monitor oder auf einer Tafel nach und nach mit jeweils unterschiedlicher Parametrisierung, das heißt mit unterschiedlichen Werten eines oder mehrerer Parameter, beispielsweise mit unterschiedlichen Werten der Bildgröße und/oder des Kontrasts präsentiert. Der reale Proband hat zu jeder ihm präsentierten Parametrisierung des Stimulusbilds sein subjektives Erkennen oder Nichterkennen eines auf dem Stimulusbild gezeigten Features mitzuteilen. Diese subjektive Antwort des realen Probanden kann beispielsweise durch ein Betätigen einer Taste oder eines Knopfs erfolgen, vorzugsweise einer bestimmten Taste für Erkennen und einer bestimmten anderen Taste für Nichterkennen. Vorzugsweise muss der Proband in jedem Fall eine konkrete Entscheidung treffen, das heißt er hat sich beispielsweise entweder für "erkannt" oder "nicht erkannt" zu entscheiden. Im Fall eines Landoltrings ist vorzugsweise vorgesehen, dass sich der Proband für eine konkrete Lückenausrichtung aus den möglichen Winkeln "0°", "45°", "90°", "135°", "180°", "225°", "270°" oder "315°" zu entscheiden hat. Notfalls hat der Proband also zu raten. Diese für den Probanden erforderliche Entscheidung wird auch mit *forced choice paradigm* (zu deutsch: "Erzwungene Wahl") bezeichnet.

Die für den ersten realen Probanden beschriebene Vorgehensweise wird bei einer Vielzahl von weiteren realen Probanden durchgeführt. Dabei ist es wichtig, durch standardisierte Bedingungen bei der Datenerhebung eine Vergleichbarkeit der jeweils gewonnenen Daten sicherzustellen. Vorzugsweise sind der Abstand zwischen Proband und Monitor bzw. Tafel, die Ausleuchtung und/oder die Expositionszeit standardisiert. Auch die einzelnen Probanden sind vorzugsweise möglichst gleichartig. Beispielsweise können alle Probanden junge gesunde Menschen ohne Fehlsichtigkeit mit normalen neuronalen Eigenschaften sein. Vorzugsweise können alle Probanden eine Fehlsichtigkeit aufweisen. Die unterschiedlich parametrisierten Stimulusbilder können den Probanden jeweils präsentiert werden, während diese ein optisches Hilfsmittel benutzen oder nicht benutzen.

Der Trainingsdatensatz kann aber auch verschiedene Gruppen von realen Probanden umfassen, beispielsweise Kinder und Erwachsene; Erwachsene und Senioren; bzw. Kinder, Erwachsene und Senioren. Darüber hinaus kann der Trainingsdatensatz verschiedene Altersgruppen umfassen, beispielsweise 30 bis 40 Jährige, 40 bis 50 Jährige, 50 bis 60 Jährige und 60 bis 70 Jährige.

Weiterhin können jedem einzelnen realen Probanden auch mehrere Stimulusbilder mit unterschiedlichen Features präsentiert werden. Beispielsweise können bei einem Einsatz des Landoltrings als Stimulusbild die acht verschiedenen Lückenausrichtungen des C als verschiedene Feature-Ausprägungen präsentiert werden. Dabei können die einzelnen Ausrichtungen jeweils blockweise mit variierender Parametrisierung oder beliebig gemischt bzw. im ständigen Wechsel miteinander präsentiert werden.

Es können beispielsweise 100 - 1.000.000 Präsentationen, vorzugsweise 1.000 - 100.000 Präsentationen, besonders bevorzugt 2.000 bis 10.000 Präsentationen eines Stimulusbilds mit einer bestimmten Feature-Ausprägung in unterschiedlichen Parametrisierungen bei der Vielzahl von Probanden insgesamt durchgeführt werden. Bei der Präsentation von Landoltringen mit acht verschiedenen Lückenausrichtungen multipliziert sich die Anzahl der Präsentationen beispielsweise mit acht.

Weiterhin können mehrere Durchläufe mit jedem einzelnen realen Probanden erfolgen, wobei das Stimulusbild in jedem Durchlauf vorzugsweise eine unterschiedlich ausgeprägte optische Aberration, beispielsweise eine unterschiedliche Schärfe aufweist. Beispielsweise kann ein erster Durchlauf mit einem scharfen Stimulusbild und ein zweiter Durchlauf mit einem unscharfen Stimulusbild erfolgen. Alternativ dazu kann bei einer Gruppe von gleichartigen realen Probanden für jeden Probanden jeweils nur ein Durchlauf zu einem Stimulusbild mit unterschiedlichen Parametrisierungen und/oder unterschiedlichen Feature-Ausprägungen durchgeführt werden; dabei kann jedoch einer ersten Anzahl von Probanden das Stimulusbild mit einer ersten Schärfe und einer zweiten Anzahl von Probanden das Stimulusbild mit einer zweiten Schärfe bereitgestellt werden. Optional kann dabei einer dritten Anzahl von Probanden das Stimulusbild mit einer dritten Schärfe bis hin zu einer n-ten Anzahl von Probanden das Stimulusbild mit einer n-ten Schärfe bereitgestellt werden. Beispielsweise kann bei einer Gruppe von gleichartigen realen Probanden ein Teil der Probanden für die beschriebene Bestimmung der subjektiven Sehleistung jeweils ein scharfes Stimulusbild und ein anderer Teil der Probanden jeweils ein unscharfes Stimulusbild präsentiert bekommen.

Vorzugsweise können jedem einzelnen Probanden die Stimulusbilder mit fünf bis zehn verschiedenen Abstufungen der Schärfe präsentiert werden. Entsprechend resultiert beispielsweise eine Gesamtzahl von 4.000 - 80.000.000 Präsentationen, vorzugsweise von 40.000 - 8.000.000 Präsentationen, besonders bevorzugt von 80.000 - 800.000 Präsentationen für die Erstellung eines Trainingsdatensatzes.

Die bereitgestellten Trainingsdatensätze umfassen die unterschiedlich parametrisierten und gegebenenfalls mit unterschiedlichen Feature-Ausprägungen versehenen Stimulusbilder sowie gegebenenfalls Informationen zu den tatsächlichen Feature-Ausprägungen der einzelnen Stimulusbilder. Dabei besteht eine eindeutige Zuordnung zwischen jedem einzelnen Stimulusbild und dessen tatsächlicher Feature-Ausprägung. Außerdem umfassen die Trainingsdatensätze vorzugsweise die jeweilige subjektive Antwort der Probanden zum Erkennen des bzw. der auf den Stimulusbildern gezeigten Features. Alternativ zur Abfrage subjektiver Sehleistung bei realen Probanden kann der Trainingsdatensatz auch ausschließlich synthetisch erzeugt sein. In diesem Fall umfasst der Trainingsdatensatz also nur die unterschiedlich parametrisierten und mit unterschiedlichen Feature-Ausprägungen versehenen Stimulusbilder.

Das neuronale Netzwerk ist vorzugsweise aus verschiedenen Schichten aufgebaut. Es kann eine Eingangsschicht, eine bestimmte Anzahl von verborgenen Schichten sowie eine Ausgabeschicht umfassen. Jede Schicht umfasst dabei eine Vielzahl von Neuronen. Die Neuronen benachbarter Schichten sind dabei unter Berücksichtigung von Gewichten miteinander verbunden. Der Trainingsdatensatz wird vorzugsweise der Eingangsschicht bereitgestellt. Dabei werden der Eingangsschicht insbesondere die einzelnen Stimulusbilder und gegebenenfalls die Informationen über die auf den einzelnen Stimulusbilder jeweils tatsächlich enthaltene Feature-Ausprägung bereitgestellt. Weiterhin kann der Eingangsschicht die jeweilige subjektive Antwort der Probanden zum Erkennen des bzw. der auf den Stimulusbildern gezeigten Features bereitgestellt werden. Beim Training des neuronalen Netzwerks gemäß Schritt b) werden insbesondere die Gewichte so angeglichen, dass eine korrekte Zuordnung zwischen den einzelnen Stimulusbildern und den tatsächlich jeweils auf diesen enthaltenen Feature-Ausprägungen in der Ausgabeschicht zustande kommt. Der Trainingsvorgang durchläuft vorzugsweise mehrere Wiederholungen. Dabei wird anhand der auftretenden Änderungen der Gewichte eine Lernrate bestimmt. Die Anzahl von Wiederholungen wird vorzugsweise mittels der Lernrate bestimmt. Der Trainingsvorgang kann beispielsweise abgebrochen werden, wenn die Lernrate einen bestimmten unteren Grenzwert erreicht oder unterschreitet. Das neuronale Netzwerk wird in diesem Fall als ausreichend trainiert angesehen.

Unter dem generischen Erstellen gemäß Schritt c) versteht sich ein Generieren bzw. Auswählen des Stimulusbilds an sich. Als Stimulusbild kann beispielsweise ein Landoltring (oder "Landolt-C"), ein Snellen-Haken (oder "E-Haken"), ein Buchstabe, eine Zahl, ein Kontrastmuster oder ein natürliches Bild vorgesehen werden. Das generische Erstellen des Stimulusbilds umfasst vorzugsweise das Vorsehen eines mehrere Feature-Ausprägungen umfassenden Feature-Raums, d.h. der Möglichkeit der Auswahl unterschiedlicher Feature-Ausprägungen, beispielsweise der acht möglichen Ausrichtungen der Lücke beim Landoltring. Der Feature-Raum kann ein- oder mehrdimensional sein.

Das im Schritt c) generisch erstellte Stimulusbild und/oder der Feature-Raum entsprechen vorzugsweise dem bei der Erstellung des zumindest einen Trainingsdatensatzes verwendeten Stimulusbild und/oder Feature-Raum. Vorzugsweise werden die Schritte a), b) und c) zeitlich in dieser Reihenfolge durchgeführt. Alternativ dazu ist auch die Auswahl eines Trainingsdatensatzes passend zum generisch erstellten Stimulusbild und dessen Feature-Raum denkbar. Der Trainingsdatensatz könnte beispielsweise von einer Datenbank aus einer Vielzahl von Trainingsdatensätzen ausgewählt werden. In diesem Fall werden die Schritte a), b) und c) also in der zeitlichen Reihenfolge c) -> a) -> b) durchgeführt.

Vorzugsweise wird im Schritt d) eine der Feature-Ausprägungen aus dem Feature-Raum ausgewählt und im Stimulusbild vorgesehen. Beispielsweise wird beim Landoltring eine der acht möglichen Ausrichtungen der Lücke als Feature-Ausprägung ausgewählt und im Stimulusbild vorgesehen. Die Auswahl und das Vorsehen der Feature-Ausprägung erfolgt vorzugsweise vor dem Parametrisieren des Stimulusbilds. Die Feature-Ausprägung wird vorzugsweise zufällig ausgewählt, beispielsweise anhand einer Normalverteilung oder Gleichverteilung. Im Fall eines mehrdimensionalen Feature-Raum kann die Feature-Ausprägung vektorförmig verstanden werden. Die Auswahl und das Vorsehen der Feature-Ausprägung erfolgt vorzugsweise durch eine Steuersoftware. Die Steuersoftware kann einen psychophysischen Algorithmus verwenden oder ein psychophysischer Algorithmus sein.

Das Parametrisieren nach Schritt d) erfolgt vorzugsweise durch die Steuersoftware bzw. durch den psychophysischen Algorithmus. Beim Parametrisieren nach Schritt d) werden vorzugsweise Eingangswerte (das heißt Anfangswerte) für die Steuersoftware bzw. für den psychophysischen Algorithmus festgelegt.

Die erste Parametrisierung wird im Sinn dieser Anmeldung als konkreter Wert bzw. konkrete Werte eines bzw. mehrerer betreffender ersten Parameter verstanden. Der bzw. die ersten Parameter bilden einen ersten Parameterraum. Allgemeiner gesprochen ist die erste Parametrisierung ein konkreter Punkt im ein- oder mehrdimensionalen ersten Parameterraum. Handelt es sich bei dem betrachteten ersten Parameter beispielsweise um die Bildgröße, so ist die erste Parametrisierung ein konkreter Wert der Bildgröße. Die Bildgröße entspricht einem sich aus ihr bei Standard-Bedingungen ergebenden Sehwinkel. Der Stimuluskontrast ist ein Kontrastwert des Stimulusbilds an sich oder der Kontrastwert eines auf dem Stimulusbild gezeigten Features. Der Stimuluskontrast kann ein schwarz-weiß-Kontrast und/oder ein Farbkontrast sein. Die Stimulusform ist die konkrete Form eines auf dem Stimulusbild gezeigten Features. Beispielsweise kann das Stimulusbild als Feature einen Kreis umfassen. In diesem Fall kann die erste Parametrisierung ein auf den Kreis aufmodulierter Sinus sein, wodurch der Kreis mit Ausbuchtungen versehen werden kann. Der Kreis kann von einer Person als perfekter Kreis oder als deformiert wahrgenommen werden (bzw. vom neuronalen Netzwerk entsprechend als perfekter Kreis oder als deformiert klassifiziert werden). Der Parameter Form kann auch beispielsweise durch das Vorsehen von Radialfrequenzmustern, engl. *radial frequency patterns,* als Stimulusbilder untersucht werden. Gemäß dem erfindungsgemäßen Verfahren kann die erste Parametrisierung beispielsweise ausschließlich durch die Bildgröße erfolgen. Alternativ dazu kann die erste Parametrisierung ausschließlich durch den Stimuluskontrast erfolgen, wobei die Stimulusbilder in ihrer Bildgröße unverändert bleiben. Weiterhin kann die erste Parametrisierung ausschließlich durch die Stimulusform erfolgen, wobei die Stimulusbilder in ihrer Bildgröße unverändert bleiben.

Die zweite Parametrisierung erfolgt durch das Anwenden von zumindest einer Eigenschaft des Auges der Person und/oder von zumindest einer Eigenschaft eines optischen Hilfsmittels (beispielsweise eines Brillenglases), vorzugsweise durch das Anwenden einer optischen Aberration oder mehrerer optischer Aberrationen des Auges und/oder des optischen Hilfsmittels (des Brillenglases), auf das Stimulusbild. Die zumindest eine Eigenschaft ist also vorzugsweise zumindest eine optische Aberration. Die optische Aberration bzw. die optischen Aberrationen des Auges und/oder des optischen Hilfsmittels können Defokus, Astigmatismus, Koma und/oder Aberrationen höherer Ordnung umfassen. Eine optische Aberration kann beispielsweise durch einen Brechwert des Auges und/oder des optischen Hilfsmittels in der Maßeinheit Dioptrie (dpt) bemessen werden. Das optische Hilfsmittel (beispielsweise das Brillenglas) bewirkt vorzugsweise eine Korrektion, das heißt einen Ausgleich einer Fehlsichtigkeit des Auges. Dabei ist eine optische Aberration des Auges durch eine entsprechende optische Aberration am optischen Hilfsmittel auszugleichen. Dabei weist die optische Aberration des optischen Hilfsmittels vorzugsweise das umgekehrte Vorzeichen wie die optische Aberration des Auges auf. Beispielsweise ist die optische Aberration des Auges +1 dpt Defokus und die optische Aberration des optischen Hilfsmittels -1 dpt Defokus.

Die zweite Parametrisierung wird im Sinn dieser Anmeldung als konkreter Wert bzw. konkrete Werte eines bzw. mehrerer betreffender zweiter Parameter verstanden. Der bzw. die zweiten Parameter bilden einen zweiten Parameterraum. Allgemeiner gesprochen ist die zweite Parametrisierung ein konkreter Punkt im ein- oder mehrdimensionalen zweiten Parameterraum. Handelt es sich bei dem betrachteten zweiten Parameter beispielsweise um eine Schärfe des Stimulusbilds, so ist die zweite Parametrisierung ein konkreter Wert der Schärfe des Stimulusbilds. In diesem Beispiel korrespondiert der Brechwert als Eigenschaft des Auges und/oder des optischen Hilfsmittels (insbesondere des Brillenglases) zur Schärfe als zweite Parametrisierung. Die zweite Parametrisierung kann alternativ dazu oder zusätzlich durch einen Kontrast erfolgen. Auch in diesem Beispiel korrespondiert beispielsweise der Brechwert als Eigenschaft des Auges und/oder des optischen Hilfsmittels (insbesondere des Brillenglases) zum Kontrast als zweite Parametrisierung. Dieses Beispiel zeigt außerdem, dass der Parameter Kontrast je nach Ausgestaltung des Verfahrens dem ersten und/oder dem zweiten Parameterraum angehören kann. Diese Aussage lässt sich verallgemeinern. Im Sinne dieser Anmeldung wird jedoch klar zwischen ersten und zweiten Parametern unterschieden: Erste Parameter werden zur Bestimmung der Schwelle der subjektiven Sehleistung eingesetzt, das heißt die Schwelle der subjektiven Sehleistung wird durch Variieren der ersten Parametrisierung bestimmt. Die erste Parametrisierung entspricht also einer Reizstärke. Zweite Parameter drücken die Auswirkung unterschiedlicher Eigenschaften optischer Systeme auf ein Stimulusbild aus.

Die erste und zweite Parametrisierung betreffen vorzugsweise verschiedene Parameter, beispielsweise die erste Parametrisierung die Bildgröße und die zweite Parametrisierung eine optische Aberration.

Optische Aberrationen können allgemein Wellenfrontfehler sein, die in einer Beschreibung von Zernike-Koeffizienten oder aber auch in jeder anderen Beschreibung von optischen Fehlern vorliegen. Diese können dabei zentral oder auch peripher am Auge und/oder am optischen Hilfsmittel (insbesondere am Brillenglas) gemessen oder berechnet sein. Zusätzlich oder alternativ zur optischen Aberration kann die zumindest eine Eigenschaft auch zumindest ein Skotom (vollständiger oder teilweiser Sensibilitätsverlust bei einem Teilbereich des Gesichtsfelds, z.B. bedingt durch altersbedingte Makuladegeneration oder diabetische Retinopathie), eine Verzerrung und/oder eine Trübung sein. Auch eine oder mehrere dieser Eigenschaften können auf das Stimulusbild angewendet werden. Als zweite Parametrisierung korrespondiert beispielsweise zur Trübung ein Kontrast bzw. eine Kontraständerung, zu einem Skotom eine lokale Verdeckung bzw. ein fehlender Bereich im Stimulusbild und zu einer Verzerrung eine Formänderung.

Die Erkennungswahrscheinlichkeitsfunktion ordnet jeder ersten Parametrisierung eine Erkennungswahrscheinlichkeit zu. Bei der Erkennungswahrscheinlichkeitsfunktion handelt es sich vorzugsweise um eine Sigmoidfunktion. Die Sigmoidfunktion wird wegen ihres Verlaufs auch Schwanenhalsfunktion oder S-Funktion genannt. Mathematisch lässt sich eine Sigmoidfunktion beispielsweise durch ein Kumulieren von Gaußverteilungen bestimmen.

Unter Klassifizieren des Stimulusbilds (siehe Schritt f) und Schritt i)) versteht man die Zuordnung des Stimulusbilds zu verschiedenen Kategorien. Die Klassifizierung des Stimulusbilds durch das trainierte neuronale Netzwerk kann beispielsweise "erkannt", "nicht erkannt" oder (im Fall eines Landoltrings) "0°", "45°", "90°", "135°", "180°", "225°", "270°" oder "315°" sein. Die durch das trainierte neuronale Netzwerk erfolgte Klassifizierung kann durch die Steuersoftware (vorzugsweise durch den psychophysischen Algorithmus) mit einer tatsächlich vorhandenen Feature-Ausprägung, also beispielsweise mit der tatsächlich vorhandenen Lückenausrichtung im Fall des Landoltrings verglichen werden. Insbesondere kann die Steuersoftware (vorzugsweise der psychophysische Algorithmus) die Klassifizierung bei "erkannt" bzw. bei korrekter Bestimmung der Ausrichtung der Lücke (im Fall eines Landoltrings) als positiv bestimmen und bei "nicht erkannt" bzw. bei falscher Bestimmung der Ausrichtung der Lücke (im Fall eines Landoltrings) als negativ bestimmen.

Bei positiver Klassifizierung kann die Steuersoftware (vorzugsweise der psychophysische Algorithmus) im Schritt g) die Erkennungswahrscheinlichkeit für die erste Parametrisierung als 100% bestimmen. Bei negativer Klassifizierung kann die Steuersoftware (vorzugsweise der psychophysische Algorithmus) im Schritt g) die Erkennungswahrscheinlichkeit für die erste Parametrisierung als 0% bestimmen.

Vorzugsweise wird die Erkennungswahrscheinlichkeit im Schritt g) als Wert der Erkennungswahrscheinlichkeitsfunktion für die im Schritt d) vorgenommene erste Parametrisierung auf der Grundlage der Klassifizierung des Stimulusbilds bestimmt. Dazu kann die Erkennungswahrscheinlichkeitsfunktion durch ein Anfitten an die zur ersten Parametrisierung bestimmte Erkennungswahrscheinlichkeit (beispielsweise die 0% bzw. 100% aus dem vorangehenden Beispiel) angepasst werden. Als Erkennungswahrscheinlichkeit für die erste Parametrisierung wird für den weiteren Ablauf des erfindungsgemäßen Verfahrens vorzugsweise der durch die gefittete Erkennungswahrscheinlichkeitsfunktion zur ersten Parametrisierung zugeordnete Wert verwendet.

Vorzugsweise wird im Schritt h) erneut eine der Feature-Ausprägungen aus dem Feature-Raum ausgewählt und im Stimulusbild vorgesehen. Beispielsweise wird beim Landoltring eine der acht möglichen Ausrichtungen der Lücke als Feature-Ausprägung ausgewählt und im Stimulusbild vorgesehen. Die Auswahl und das Vorsehen der Feature-Ausprägung erfolgt vorzugsweise vor dem Anpassen der Parametrisierung. Die Feature-Ausprägung wird vorzugsweise zufällig ausgewählt, beispielsweise anhand einer Normalverteilung oder Gleichverteilung. Im Fall eines mehrdimensionalen Feature-Raums kann die Feature-Ausprägung vektorförmig verstanden werden.

Das Anpassen der ersten Parametrisierung des Stimulusbilds beim Schritt h) erfolgt vorzugsweise durch die Steuersoftware (insbesondere durch den psychophysischen Algorithmus) auf der Grundlage der im Schritt g) bzw. im Schritt j) bestimmten Erkennungswahrscheinlichkeit. Das Anpassen der ersten Parametrisierung des Stimulusbilds kann dabei vorzugsweise auf der Grundlage der mittels der bestimmten Erkennungswahrscheinlichkeit gefitteten Erkennungswahrscheinlichkeitsfunktion erfolgen. Daher kann sich im Folgenden der Begriff "Erkennungswahrscheinlichkeit" sowohl auf die im Schritt g) bzw. im Schritt j) bestimmte Erkennungswahrscheinlichkeit als auch auf den Wert der gefitteten Erkennungswahrscheinlichkeitsfunktion für die betreffende erste Parametrisierung beziehen. Vorzugsweise wird die erste Parametrisierung bei einer Erkennungswahrscheinlichkeit von über 50% (bzw. von zumindest 50%) derart angepasst, dass ein Erkennen des Stimulusbilds mit der neuen ersten Parametrisierung schwieriger wird, also beispielweise die Bildgröße verringert wird. Entsprechend kann die erste Parametrisierung bei einer Erkennungswahrscheinlichkeit von unter 50% derart angepasst werden, dass ein Erkennen des Stimulusbilds mit der neuen ersten Parametrisierung einfacher wird, also beispielweise die Bildgröße erhöht wird. Vorzugsweise ist auch der Betrag der Differenz zwischen der neuen ersten Parametrisierung und der vorhergehenden ersten Parametrisierung von der

Erkennungswahrscheinlichkeit abhängig. Vorzugsweise ist der Betrag dieser Differenz umso kleiner, je näher die Erkennungswahrscheinlichkeit bei 50% liegt. Das Ziel des Anpassens der ersten Parametrisierung des Stimulusbilds besteht darin, die erste Parametrisierung der Sehschwelle anzunähern.

Je nach Art des vorzugsweise verwendeten psychophysischen Algorithmus kann vorgesehen sein, dass beim Anpassen der ersten Parametrisierung im Schritt h) immer eine erste Parametrisierung eingestellt wird, die zuvor noch nicht eingestellt war, oder dass eine bereits zuvor verwendete erste Parametrisierung im Schritt h) erneut eingestellt werden darf. Entsprechend kann eine bestimmte erste Parametrisierung je nach Art des psychophysischen Algorithmus nur einmal oder mehrmals abgefragt werden. Unter "eine Abfrage" wird hier das Durchlaufen der Schritte f) und g) bzw. der Schritte i) und j) verstanden.

Im Schritt j) bestimmt vorzugsweise die Steuersoftware (insbesondere der psychophysische Algorithmus) die Erkennungswahrscheinlichkeit für die im Schritt h) angepasste erste Parametrisierung auf der Grundlage der Klassifizierung aus dem Schritt i). Dabei kann die Steuersoftware (vorzugsweise der psychophysische Algorithmus) die Erkennungswahrscheinlichkeit für die angepasste erste Parametrisierung bei positiver Klassifizierung als 100% bestimmen, sofern die betreffende erste Parametrisierung erstmals abgefragt wurde, oder beispielsweise einen Mittelwert aus 100% und der bereits in einem vorangehenden Durchgang beim Schritt j) oder bereits beim Schritt g) zur gleichen ersten Parametrisierung ermittelten Erkennungswahrscheinlichkeit bestimmen, sofern die betreffende erste Parametrisierung bereits abgefragt wurde. Unter einem Durchgang wird dabei das Durchlaufen der Schritte d) bis g) oder das einmalige Durchlaufen der Schritte h) bis j) verstanden. Bei negativer Klassifizierung kann die Steuersoftware im Schritt j) die Erkennungswahrscheinlichkeit für die angepasste erste Parametrisierung als 0% bestimmen, sofern die betreffende erste Parametrisierung erstmals abgefragt wurde, oder beispielsweise einen Mittelwert aus 0% und der bereits in einem vorangehenden Durchgang beim Schritt j) oder bereits beim Schritt g) zur gleichen ersten Parametrisierung ermittelten Erkennungswahrscheinlichkeit bestimmen, sofern die betreffende erste Parametrisierung bereits abgefragt wurde. Die Mittelung kann jeweils derart erfolgen, dass jede Abfrage des Parameterwerts die gleiche Gewichtung erhält.

Vorzugsweise wird die Erkennungswahrscheinlichkeit im Schritt j) als Wert der Erkennungswahrscheinlichkeitsfunktion für die im Schritt h) angepasste erste Parametrisierung auf der Grundlage der Klassifizierung des angepassten Stimulusbilds bestimmt. Dazu kann die Erkennungswahrscheinlichkeitsfunktion durch ein Anfitten an die zur ersten Parametrisierung bestimmte Erkennungswahrscheinlichkeit (beispielsweise die 0% bzw. 100% aus dem vorangehenden Beispiel) angepasst werden. Die Erkennungswahrscheinlichkeitsfunktion ist vorzugsweise eine an die zur angepassten ersten Parametrisierung bestimmte Erkennungswahrscheinlichkeit sowie an gegebenenfalls in vorangehenden Durchgängen beim Schritt j) bzw. beim Schritt g) zu den jeweiligen ersten Parametrisierungen bestimmte Erkennungswahrscheinlichkeiten angefittete Sigmoidfunktion. Als Erkennungswahrscheinlichkeit für die erste Parametrisierung wird für den weiteren Ablauf des erfindungsgemäßen Verfahrens vorzugsweise der durch die gefittete Erkennungswahrscheinlichkeitsfunktion zur ersten Parametrisierung zugeordnete Wert verwendet.

Im Schritt k) überprüft vorzugsweise die Steuersoftware (insbesondere der psychophysische Algorithmus), ob ein Abbruchkriterium erreicht ist. Falls ein Abbruchkriterium erreicht ist, führt die Steuersoftware den Schritt I) aus. Falls kein Abbruchkriterium erreicht ist, werden erneut die Schritte h) bis k) wiederholt. Der psychophysische Algorithmus führt solange eine Rekursion durch, bis die Schwelle der subjektiven Sehleistung aufgefunden ist, das heißt bis die erste Parametrisierung (insbesondere die Bildgröße) sich hinreichend der Schwelle genähert hat und/oder die Schwelle erreicht hat, so dass nachfolgend im Schritt I) die Schwelle bestimmt werden kann.

Beim letztmaligen gemäß Schritt k) durchgeführten Wiederholen der Schritte h) bis j) kann bei bestimmten Ausführungsformen der Erfindung bereits nach dem Schritt h) oder i) abgebrochen werden. Je nach Ausführungsform kann beim letztmaligen Wiederholen bereits das Anpassen der ersten Parametrisierung des Stimulusbilds für die Bestimmung der Schwelle im Schritt I) genügen (siehe unten). Die Durchführung der Schritte i) und/oder j) ist beim letztmaligen Wiederholen also optional.

Der vorzugsweise verwendete psychophysische Algorithmus kann nach einem adaptiven Stufenverfahren vorgehen. Geeignete Beispiele für das adaptive Stufenverfahren sind BestPEST, PSI, das up-down-Staircase-Verfahren und das constant-stimuli-Verfahren. Je nach eingesetztem Verfahren kann sich die Vorgehensweise beim Bestimmen der Schwelle der subjektiven Sehleistung gemäß Schritt I) unterscheiden. Beispielweise kann die Schwelle der subjektiven Sehleistung (wie z. B. beim up-down-staircase-Verfahren) durch Mittelung der an mehreren Umkehrpunkten erreichten Werte der ersten Parametrisierung bestimmt werden. Unter Umkehrpunkten (bzw. lokalen Extrema) werden hier Punkte in einer graphischen Auftragung der ersten Parametrisierung über die fortlaufende Zahl der Durchgänge verstanden, an denen ein Wechseln der Anpassung von kleineren hin zu größeren ersten Parametrisierungen oder von größeren hin zu kleineren ersten Parametrisierungen stattfindet. Unter einem Durchgang wird das Durchlaufen der Schritte d) bis g) oder das einmalige Durchlaufen der Schritte h) bis j) verstanden. Bei anderen adaptiven Stufenverfahren (z.B. BestPEST) kann der Wert der Schwelle als die erste Parametrisierung, der die Erkennungswahrscheinlichkeitsfunktion nach Erreichen des Abbruchkriteriums die Erkennungswahrscheinlichkeit 50% zuordnet, bestimmt werden. Dabei ergibt sich die Erkennungswahrscheinlichkeitsfunktion vorzugsweise als ein Fit an die in den einzelnen Durchgängen bei den Schritten j) bzw. beim Schritt g) zu den jeweiligen ersten Parametrisierungen bestimmten Erkennungswahrscheinlichkeiten.

Vorzugsweise arbeitet die Steuersoftware (bevorzugt der psychophysische Algorithmus) mit einer sich bei jedem Durchlaufen der Schritte h - j zufällig ändernden Feature-Ausprägung, beispielsweise im Fall eines Landoltrings mit einer sich zufällig ändernden Lückenausrichtung.

Im Schritt I) wird die Schwelle der subjektiven Sehleistung vorzugsweise auf der Grundlage der zu den jeweiligen ersten Parametrisierungen des Stimulusbilds bestimmten Erkennungswahrscheinlichkeiten bestimmt. Dazu sind verschiedene Ausführungsformen möglich. Beispielsweise kann die beim letztmaligen Durchlaufen der Schritte h) bis j) erreichte erste Parametrisierung als Wert der Schwelle der subjektiven Sehleistung übernommen werden. In diesem Fall könnte beim letztmaligen Durchlaufen also auf die Schritte i) und/oder j) verzichtet werden. Vorzugsweise wird der Wert der Schwelle der subjektiven Sehleistung als die erste Parametrisierung, der die Erkennungswahrscheinlichkeitsfunktion nach Erreichen des Abbruchkriteriums die Erkennungswahrscheinlichkeit 50% zuordnet, bestimmt. Vorzugsweise ergibt sich die Erkennungswahrscheinlichkeitsfunktion als ein Fit an die in den einzelnen Durchgängen bei den Schritten j) bzw. beim Schritt g) zu den jeweiligen ersten Parametrisierungen bestimmten Erkennungswahrscheinlichkeiten.

Gemäß Schritt m) werden die Schritte d) bis I) mehrmals sequentiell durchlaufen. Die einzelnen Schritte werden dabei vorzugsweise in der vorgegebenen Reihenfolge durchlaufen.

Gemäß Schritt m) wird vorzugsweise die zumindest eine Eigenschaft des optischen Hilfsmittels, insbesondere des Brillenglases, von Durchlauf zu Durchlauf variiert. Die zumindest eine Eigenschaft ist also bei jedem Durchlaufen der Schritte d) bis I) unterschiedlich stark ausgeprägt. Dadurch lässt sich ein Vergleich zwischen verschiedenen Werten der Eigenschaft durchführen, beispielsweise zwischen verschiedenen Brechwerten eines Brillenglases. Der Brechwert ist ein Beispiel für eine Eigenschaft des optischen Hilfsmittels (insbesondere des Brillenglases), die zum Hervorrufen einer zweiten Parametrisierung auf das Stimulusbild angewendet wird. Dabei korrespondiert dem Brechwert eine Schärfe des Stimulusbilds als zweite Parametrisierung. Der Brechwert des im Schritt n) ausgewählten optimierten optischen Hilfsmittels (insbesondere Brillenglas) korrespondiert dabei also zur Schärfe (zweite Parametrisierung) des maximal scharfen Stimulusbilds.

Das beim Schritt n) ausgewählte optische Hilfsmittel (insbesondere Brillenglas) ist spezifisch für das Auge der Person optimiert.

Der Begriff "höchste subjektive Sehleistung" (oder synonym dazu "höchster subjektiver visueller Performanzwert") steht für die (je nach Parameterart) niedrigste bzw. höchste Lage der Schwelle der subjektiven Sehleistung. Je nach Parameterart kann es sich dabei um die höchste bzw. die niedrigste erste Parametrisierung handeln, bei der ein Erkennen beispielsweise noch mit 50% Erkennungswahrscheinlichkeit möglich ist. Im Fall der Bildgröße als erstem Parameter ergibt sich die höchste subjektive Sehleistung beispielsweise für die Konfiguration des optischen Hilfsmittels (insbesondere des Brillenglases), bei der im Schritt I) die niedrigste Schwelle bestimmt wurde. Für diese Konfiguration des optischen Hilfsmittels ist also der geringste Wert für die Bildgröße zum Erkennen erforderlich. Diese Konfiguration des optischen Hilfsmittels ermöglicht für das Auge der Person also das höchste Auflösungsvermögen.

Mit dem erfindungsgemäßen Verfahren kann ein für eine bestimmte Person optimiertes optisches Hilfsmittel (insbesondere ein für die bestimmte Person optimiertes Brillenglas) ohne weiteres Zutun der Person bereitgestellt werden. Insbesondere muss die Person keine langwierigen Untersuchungen mit einer Vielzahl von verschieden ausgestalteten optischen Hilfsmitteln (insbesondere mit einer Vielzahl verschieden ausgestalteter Brillengläser) über sich ergehen lassen. Damit werden störende Einflüsse z. B. durch Ermüdung vermieden. Darüber hinaus wird keine Modellierung von Filterfunktionen benötigt. Weiterhin ist es bei der Anwendung des erfindungsgemäßen Verfahrens nicht erforderlich, die Vielzahl von verschieden ausgestalteten optischen Hilfsmitteln (insbesondere Brillengläsern) zu fertigen. Mit Hilfe des erfindungsgemäßen Verfahren kann die subjektive Sehleistung der Person schnell, ohne störende Einflüsse und mit einer guten Genauigkeit bestimmt wird. Die Auswirkung von einer Vielzahl verschieden ausgestalteter optischer Hilfsmittel (insbesondere Brillengläser) auf die subjektive Sehleistung der Person kann kostengünstig und schnell verglichen werden, und somit kostengünstig und schnell eine für diese Person optimierte Eigenschaft des optischen Hilfsmittels (insbesondere Brillenglases) ausgewählt werden. Darüber hinaus kann das erfindungsgemäße Verfahren auch für Kombinationen verschiedener optischer Aberrationen eingesetzt werden. Beispielsweise können verschiedene Kombinationen von Astigmatismus und Defokus verglichen werden und eine optimale Kombination daraus ausgewählt werden.

Nach einer bevorzugten Ausführungsform der Erfindung ist vorgesehen, dass das Abbruchkriterium eines oder mehrere der folgenden Kriterien umfasst:
- Erreichen einer vorbestimmten Anzahl von Wiederholungen des Durchlaufens der Schritte h) bis j) gemäß Schritt k),
- Erreichen einer vorbestimmten Anzahl von Unterschreitungen eines vorbestimmten Grenzwerts durch eine Differenz von beim wiederholten Durchlaufen der Schritte h) bis j) gemäß Schritt k) aufeinanderfolgenden angepassten ersten Parametrisierungen,
- Erreichen einer vorbestimmten Anzahl von Reversals.

Je nach der vorbestimmten Anzahl kann nach dem zweiten hier genannten Kriterium ein einmaliges Unterschreiten zur Erfüllung des Abbruchkriteriums genügen (die vorbestimmte Anzahl ist gleich 1) oder ein zweimaliges oder häufigeres Unterschreiten zur Erfüllung des Abbruchkriteriums erforderlich sein. Dieses Kriterium beschreibt also eine (hinreichende) Konvergenz der ersten Parametrisierung mit der Schwelle der subjektiven Sehleistung. Die "Differenz von beim wiederholten Durchlaufen der Schritte h) bis j) gemäß Schritt k) aufeinanderfolgenden angepassten ersten Parametrisierungen" ist beispielsweise eine Differenz aus in aufeinanderfolgenden Durchgängen beim Schritt h) angepassten Bildgrößen bzw. den angepassten Bildgrößen entsprechenden Sehwinkeln (Δαᵥ). Maßgeblich ist der Betrag der Differenz. Es ist also gleichgültig, ob die Konvergenz zu größeren Werten der ersten Parametrisierung hin oder zu kleineren Werten der ersten Parametrisierung hin erfolgt.

"Reversals" stehen für die oben bereits beschriebenen Umkehrpunkte (bzw. lokalen Extrema) in einer Abfolge der gemäß Schritt h) angepassten ersten Parametrisierung beim Wiederholen der Schritte h) bis j) gemäß Schritt k). Der Begriff "Reversal" könnte daher auch durch den Begriff "Umkehrpunkt" ersetzt werden. Ein "Reversal" kann auch definiert werden als ein Wechsel zwischen positiver und negativer Klassifizierung beim Schritt i), das heißt zwischen den Klassifizierungen "erkannt" und "nicht erkannt", in der Abfolge der angepassten ersten Parametrisierung beim Wiederholen der Schritte h) bis j) gemäß Schritt k). Die vorbestimmte Anzahl von Reversals steht also für ein (insbesondere mehrmaliges) Hin- und Herspringen zwischen positiver und negativer Klassifizierung bzw. zwischen negativer und positiver Klassifizierung. Das Erreichen einer hinreichend hohen Anzahl (also insbesondere der vorbestimmten Anzahl) von Reversals ist ein Anzeichen dafür, dass die erste Parametrisierung genau um den Wert der Schwelle pendelt. Das heißt, dass die Schwelle wertmäßig genau zwischen den an den Umkehrpunkten erreichten ersten Parametrisierungen liegt. Vorzugsweise wird der Wert der Schwelle als der Mittelwert aus den an den Umkehrpunkten erreichten ersten Parametrisierungen bestimmt.

Vorzugsweise hängt die Vorgehensweise zur wertmäßigen Bestimmung der Schwelle von der Art des Abbruchkriteriums ab. Bei dem Erreichen der vorbestimmten Anzahl von Wiederholungen des Durchlaufens der Schritte h) bis j) als Abbruchkriteriums kann beispielsweise beim letztmaligen Durchlaufen erreichte erste Parametrisierung als Wert der Schwelle übernommen werden. In diesem Fall könnte beim letztmaligen Durchlaufen also auf die Schritte i) und j) verzichtet werden. Vorzugsweise wird der Wert der Schwelle als die erste Parametrisierung, der die Erkennungswahrscheinlichkeitsfunktion nach Erreichen des Abbruchkriteriums die Erkennungswahrscheinlichkeit 50% zuordnet, bestimmt. Vorzugsweise ergibt sich die Erkennungswahrscheinlichkeitsfunktion als ein Fit an die in den einzelnen Durchgängen bei den Schritten j) bzw. beim Schritt g) zu den jeweiligen ersten Parametrisierungen bestimmten Erkennungswahrscheinlichkeiten.

Nach einer weiteren bevorzugten Ausführungsform der Erfindung ist vorgesehen, dass die beim Parametrisieren des Stimulusbilds nach Schritt d) vorgenommene zweite Parametrisierung das Anwenden zumindest einer Eigenschaft eines Auges einer Person, insbesondere einer optischen Aberration des Auges der Person, auf das Stimulusbild umfasst.

Wie oben bereits dargelegt, wird das erfindungsgemäße Optimieren eines optischen Hilfsmittels (insbesondere eines Brillenglases) spezifisch für ein Auge einer bestimmten Person durchgeführt. Dank des erfindungsgemäßen Verfahrens ist es nicht erforderlich, dass die Person langwierige subjektive Messungen über sich ergehen lassen muss.

Vorzugsweise gehört die Person nicht den realen Probanden an, die zur Erstellung des zumindest einen Trainingsdatensatzes beigetragen haben.

Die zweite Parametrisierung kann also die Anwendung einer optischen Aberration des Auges der Person auf das Stimulusbild umfassen. Die zweite Parametrisierung kann auch die Anwendung verschiedener optischer Aberrationen des Auges der Person auf das Stimulusbild umfassen. Die zweite Parametrisierung kann alternativ oder zusätzlich die Anwendung von Verzerrungen und/oder Skotomen des Auges der Person auf das Stimulusbild umfassen.

Nach einer weiteren bevorzugten Ausführungsform der Erfindung ist vorgesehen, dass die nach Schritt m) beim Parametrisieren des Stimulusbilds nach Schritt d) auf das Stimulusbild angewandte Eigenschaft des optischen Hilfsmittels eine optische Aberration des optischen Hilfsmittels umfasst. Das optische Hilfsmittel ist dabei vorzugsweise ein Brillenglas.

Die zweite Parametrisierung kann also die Anwendung einer optischen Aberration des optischen Hilfsmittels (insbesondere des Brillenglases) auf das Stimulusbild umfassen. Die zweite Parametrisierung kann auch die Anwendung verschiedener optischer Aberrationen des optischen Hilfsmittels (insbesondere des Brillenglases) auf das Stimulusbild umfassen. Die beim Durchlaufen der Schritte d) bis I) im Stimulusbild parametrisierten optischen Aberrationen können also vom Auge der Person und/oder vom optischen Hilfsmittel resultieren. Die zweite Parametrisierung bezieht sich also vorzugsweise auf das gesamte optische System, das heißt auf das Auge und/oder auf ein optisches Hilfsmittel. Das optische Hilfsmittel (beispielsweise das Brillenglas) bewirkt vorzugsweise eine Korrektion, das heißt einen Ausgleich einer Fehlsichtigkeit des Auges. Dabei ist eine optische Aberration des Auges durch eine entsprechende optische Aberration am optischen Hilfsmittel auszugleichen. Dabei weist die optische Aberration des optischen Hilfsmittels vorzugsweise das umgekehrte Vorzeichen wie die optische Aberration des Auges auf. Beispielsweise ist die optische Aberration des Auges +1 dpt Defokus und die optische Aberration des optischen Hilfsmittels -1 dpt Defokus.

Nach einer weiteren bevorzugten Ausführungsform der Erfindung ist vorgesehen, dass die optische Aberration des Auges der Person gemessen und/oder berechnet ist, und/oder dass die optische Aberration des optischen Hilfsmittels gemessen und/oder berechnet ist. Selbstverständlich kann auch vorgesehen sein, dass mehrere optische Aberrationen des Auges der Person gemessen und/oder berechnet sind, bzw. dass mehrere optische Aberrationen des optischen Hilfsmittels gemessen und/oder berechnet sind. Das optische Hilfsmittel ist vorzugsweise ein Brillenglas.

Nach einer weiteren bevorzugten Ausführungsform der Erfindung ist vorgesehen, dass der Schritt c) das Vorsehen eines mehrere Feature-Ausprägungen umfassenden Feature-Raums umfasst, dass im Schritt d) vor dem Parametrisieren des Stimulusbilds eine der Feature-Ausprägungen aus dem Feature-Raum ausgewählt und im Stimulusbild vorgesehen wird, und dass im Schritt h) vor dem Anpassen der ersten Parametrisierung erneut eine der Feature-Ausprägungen aus dem Feature-Raum ausgewählt und im Stimulusbild vorgesehen wird.

Das Vorsehen des Feature-Raums schafft also die Möglichkeit der Auswahl unterschiedlicher Feature-Ausprägungen. Der Feature-Raum kann ein- oder mehrdimensional sein. Handelt es sich beim Stimulusbild um einen Landoltring, so umfasst der Feature-Raum beispielsweise die acht möglichen Ausrichtungen der Lücke des Landoltrings. In diesem Beispiel wird im Schritt d) eine der acht möglichen Ausrichtungen der Lücke als Feature-Ausprägung ausgewählt und im Stimulusbild vorgesehen. Weiterhin wird in diesem Beispiel im Schritt h) erneut eine der acht möglichen Ausrichtungen der Lücke als Feature-Ausprägung ausgewählt und im Stimulusbild vorgesehen. Die Feature-Ausprägung wird vorzugsweise jeweils zufällig ausgewählt, beispielsweise anhand einer Normalverteilung oder Gleichverteilung. Im Fall eines mehrdimensionalen Feature-Raums kann die Feature-Ausprägung vektorförmig verstanden werden.

Nach einer weiteren bevorzugten Ausführungsform der Erfindung ist vorgesehen, dass das neuronale Netzwerk ein Convolutional Neural Network ist.

Ein Convolutional Neural Network (Abkürzung CNN, zu deutsch etwa "faltendes neuronale Netzwerk") steht für ein neuronales Netzwerk, das eine Netzwerkarchitektur mit Faltungsneuronen aufweist. Ein Convolutional Neural Network ist besonders vorteilhaft, wenn wie beim Lernen von Bildinhalten Intensitätsverteilungen herangezogen werden.

Nach einer weiteren bevorzugten Ausführungsform der Erfindung ist vorgesehen, dass die Schritte b), c), d), e), g), h), j), k), I), m) und/oder n) durch eine Steuersoftware durchgeführt werden. Die Steuersoftware kann einen psychophysischen Algorithmus verwenden. In diesem Fall werden die Schritte d), e), g), h), j), k) und/oder I) vorzugsweise durch den psychophysischen Algorithmus durchgeführt. Besonders bevorzugt werden die Schritte d), g), h), j), k) und I) durch den psychophysischen Algorithmus durchgeführt. Im Schritt e) wird die Erkennungswahrscheinlichkeitsfunktion vorzugsweise an den psychophysischen Algorithmus bereitgestellt. Vorzugsweise werden weiterhin das optional im Schritt d) vorgenommene Auswählen einer der Feature-Ausprägungen aus dem Feature-Raum und Vorsehen im Stimulusbild sowie das optional im Schritt h) erneut vorgenommene Auswählen einer der Feature-Ausprägungen aus dem Feature-Raum und Vorsehen im Stimulusbild durch den psychophysischen Algorithmus durchgeführt. Außerdem kann in den Schritten f) und/oder i) eine positive bzw. negative Klassifizierung durch den psychophysischen Algorithmus bestimmt werden.

Weiterhin kann die Steuersoftware mehrere Teile umfassen, nämlich eine Trainingssoftware, eine Schwellenbestimmungssoftware und/oder eine Optimierungssoftware. Im Schritt a) wird der zumindest eine Trainingsdatensatz vorzugsweise an die Trainingssoftware bereitgestellt. Der Schritt b) wird vorzugsweise durch die Trainingssoftware ausgeführt. Vorzugsweise ist die Schwellbestimmungssoftware ein psychophysischer Algorithmus oder verwendet die Schwellbestimmungssoftware einen psychophysischen Algorithmus. Vorzugsweise werden die Schritte d), e), g), h), j), k) und/oder I) durch den psychophysischen Algorithmus bzw. durch die Schwellbestimmungssoftware durchgeführt. Besonders bevorzugt werden die Schritte d), g), h), j), k) und I) durch den psychophysischen Algorithmus bzw. durch die Schwellbestimmungssoftware durchgeführt. Im Schritt e) wird die Erkennungswahrscheinlichkeitsfunktion vorzugsweise durch die Optimierungssoftware an den psychophysischen Algorithmus bzw. an die Schwellbestimmungssoftware bereitgestellt. Vorzugsweise werden weiterhin das optional im Schritt d) vorgenommene Auswählen einer der Feature-Ausprägungen aus dem Feature-Raum und Vorsehen im Stimulusbild sowie das optional im Schritt h) erneut vorgenommene Auswählen einer der Feature-Ausprägungen aus dem Feature-Raum und Vorsehen im Stimulusbild durch den psychophysischen Algorithmus bzw. durch die Schwellbestimmungssoftware durchgeführt. Außerdem kann in den Schritten f) und/oder i) eine positive bzw. negative Klassifizierung durch den psychophysischen Algorithmus bzw. durch die Schwellbestimmungssoftware bestimmt werden. Die Schritte c), e), m) und/oder n) werden vorzugsweise durch die Optimierungssoftware durchgeführt.

Alternativ dazu kann die Steuersoftware der psychophysische Algorithmus sein. In diesem Fall werden die Schritte d), e), g), h), j), k) und/oder I) durch den psychophysischen Algorithmus, das heißt durch die Steuersoftware, durchgeführt. Vorzugsweise werden in diesem Fall die Schritte d), g), h), j), k) und I) durch den psychophysischen Algorithmus, das heißt durch die Steuersoftware, durchgeführt. Die Erkennungswahrscheinlichkeitsfunktion wird im Schritt e) vorzugsweise an den psychophysischen Algorithmus, das heißt an die Steuersoftware, bereitgestellt. Vorzugsweise werden weiterhin das optional im Schritt d) vorgenommene Auswählen einer der Feature-Ausprägungen aus dem Feature-Raum und Vorsehen im Stimulusbild sowie das optional im Schritt h) erneut vorgenommene Auswählen einer der Feature-Ausprägungen aus dem Feature-Raum und Vorsehen im Stimulusbild durch den psychophysischen Algorithmus, das heißt durch die Steuersoftware, durchgeführt. Außerdem kann in den Schritten f) und/oder i) eine positive bzw. negative Klassifizierung durch den psychophysischen Algorithmus bestimmt werden.

Die Verwendung eines psychophysischen Algorithmus ist vorteilhaft, weil durch eine adaptive Abstufung der Reizstärke (das heißt der ersten Parametrisierung) eine effiziente Bestimmung der Sehschwelle möglich ist.

Die Steuersoftware kann auch mehrere psychophysische Algorithmen umfassen. Der psychophysische Algorithmus hat das Ermitteln der Sehschwelle einer spezifischen visuellen Aufgabe zum Ziel. Dabei sind verschiedene Verfahren zur Schwellenbestimmung möglich.

Nach einer weiteren bevorzugten Ausführungsform der Erfindung ist vorgesehen, dass der psychophysische Algorithmus nach einem adaptiven Stufenverfahren vorgeht. Beispiele für ein adaptives Stufenverfahren sind BestPEST, PSI,das up-down-Staircase-Verfahren oder das constant-stimuli-Verfahren.

Erfindungsgemäß ist weiterhin ein Verfahren zum Herstellen eines erfindungsgemäß optimierten optischen Hilfsmittels vorgesehen. Das optische Hilfsmittel ist dabei vorzugsweise ein Brillenglas. Das Herstellungsverfahren umfasst die folgenden Schritte:
- Bestimmen von Oberflächenbearbeitungsdaten gemäß der in Schritt n) ausgewählten zweiten Parametrisierung des optimierten optischen Hilfsmittels,
- Zuführen der Oberflächenbearbeitungsdaten an einen Automaten zur mechanischen Bearbeitung von optischen Oberflächen zum mechanischen Bearbeiten der optischen Oberflächen von Rohlingen gemäß den Oberflächenbearbeitungsdaten.

Beispielsweise sind die Oberflächenbearbeitungsdaten auf das Vorsehen eines bestimmten Radius einer optischen Fläche eines Rohlings zum Herstellen des optimierten optischen Hilfsmittels gerichtet. Der Automat zur mechanischen Bearbeitung von optischen Oberflächen führt die mechanische Bearbeitung am Rohling gemäß den Oberflächenbearbeitungsdaten durch. Der Automat sieht also beispielsweise den bestimmten Radius einer optischen Fläche am Rohling aufgrund der Oberflächenbearbeitungsdaten vor und stellt dadurch das optimierte optische Hilfsmittel her.

Nach einer weiteren bevorzugten Ausführungsform der Erfindung ist vorgesehen, dass das mechanische Bearbeiten der optischen Oberflächen Schleifen und/oder Polieren umfasst, die Oberflächenbearbeitungsdaten Schleifdaten und/oder Polierdaten umfassen und der Automat zur mechanischen Bearbeitung von optischen Oberflächen einen Schleif- und/oder Polierautomaten umfasst.

Erfindungsgemäß ist weiterhin eine Vorrichtung zum Herstellen von optimierten optischen Hilfsmitteln vorgesehen. Die optischen Hilfsmittel sind dabei vorzugsweise Brillengläser. Die erfindungsgemäße Vorrichtung umfasst
- einen Automaten zur mechanischen Bearbeitung von optischen Oberflächen,
- eine Einrichtung zum Bestimmen von Oberflächenbearbeitungsdaten gemäß der in Schritt n) ausgewählten zweiten Parametrisierung des optimierten optischen Hilfsmittels,
- eine Auswerteeinrichtung zum Durchführen des erfindungsgemäßen Optimierungsverfahrens, umfassend Schnittstellen zum Einlesen von Trainingsdatensätzen und zur Eingabe und/oder Auswahl von Stimulusbildern sowie von Parametrisierungsdaten zu den ersten und zweiten Parametrisierungen.

Bei dem Automaten zur mechanischen Bearbeitung von optischen Oberflächen handelt es sich um eine herkömmliche Vorrichtung zur mechanischen Bearbeitung von optischen Oberflächen (insbesondere von Brillengläsern). Die mechanische Bearbeitung der optischen Oberflächen erfolgt dabei auf eine dem Fachmann bekannte Weise.

Die Auswerteeinrichtung kann dabei die Einrichtung zum Bestimmen von Oberflächenbearbeitungsdaten umfassen. Die Auswerteeinrichtung und die Einrichtung zum Bestimmen von Oberflächenbearbeitungsdaten können alternativ dazu getrennt ausgebildet sein. Es kann eine Schnittstelle zur Übermittelung der in Schritt n) ausgewählten zweiten Parametrisierung des optimierten optischen Hilfsmittels (vorzugsweise des optimierten Brillenglases) zwischen der Auswerteeinrichtung und der Einrichtung zum Bestimmen von Oberflächenbearbeitungsdaten vorgesehen sein.

Die Auswerteeinrichtung umfasst vorzugsweise einen oder mehrere Hochleistungsprozessoren und/oder Cluster. Dadurch kann die Auswerteeinrichtung die für das Trainieren des neuronalen Netzwerks erforderliche hohe Rechenleistung bereitstellen.

Vorzugsweise umfasst die erfindungsgemäße Vorrichtung weiterhin eine Testeinrichtung zur Überprüfung der bearbeiteten optimierten optischen Hilfsmittel (vorzugsweise der bearbeiteten optimierten Brillengläser).

Das erfindungsgemäße Optimierungsverfahren und/oder beliebige oben beschriebene bevorzugte Ausführungsformen davon können computerimplementiert sein. Erfindungsgemäß ist insbesondere ein Computerprogramm mit auf einem Prozessor ablauffähigem Programmcode zum Ausführen der Verfahrensschritte des erfindungsgemäßen Optimierungsverfahrens und/oder von beliebigen oben beschriebenen Ausführungsformen davon vorgesehen. Insbesondere führt das Computerprogramm die Verfahrensschritte des erfindungsgemäßen Verfahrens aus, indem es einen Algorithmus durchläuft.

Das Computerprogramm wird dazu auf einem Computer geladen oder auf einem Computer ausgeführt. Die Auswerteeinrichtung und/oder die Einrichtung zum Bestimmen von Oberflächenbearbeitungsdaten können als Computer ausgestaltet sein oder einen Computer umfassen. Alternativ kann der Computer die Auswerteeinrichtung und/oder die Einrichtung zum Bestimmen von Oberflächenbearbeitungsdaten umfassen. Der Computer weist vorzugsweise Schnittstellen zur Bedienung durch einen Benutzer und/oder zur Anzeige auf einer Anzeigeeinheit auf.

Erfindungsgemäß ist weiterhin ein nichttransitorisches Speichermedium mit einem darauf gespeicherten erfindungsgemäßen Computerprogramm vorgesehen.

Die Erfindung wird nun anhand von Ausführungsbeispielen näher erläutert. Es zeigen
- Fig. 1: ein Flussdiagramm zum Ablauf eines Ausführungsbeispiels eines erfindungsgemäßen Verfahrens zum Optimieren eines optischen Hilfsmittels,
- Fig. 2: ein Flussdiagramm zum Ablauf eines Schwellenbestimmungsverfahrens innerhalb des in Fig. 1 gezeigten Ausführungsbeispiel,
- Fig. 3A: ein Diagramm zum Ablauf eines Schwellenbestimmungsverfahrens zu einem ersten optischen Hilfsmittel,
- Fig. 3B: eine Erkennungswahrscheinlichkeitsfunktion zum ersten optischen Hilfsmittel,
- Fig. 4A: ein Diagramm zum Ablauf eines Schwellenbestimmungsverfahrens zu einem zweiten optischen Hilfsmittel,
- Fig. 4B: eine Erkennungswahrscheinlichkeitsfunktion zum zweiten optischen Hilfsmittel,
- Fig. 5: eine Gegenüberstellung von Stimulusbildern und jeweils zugehöriger Visualisierung erlernter Gewichte eines trainierten neuronalen Netzwerks.

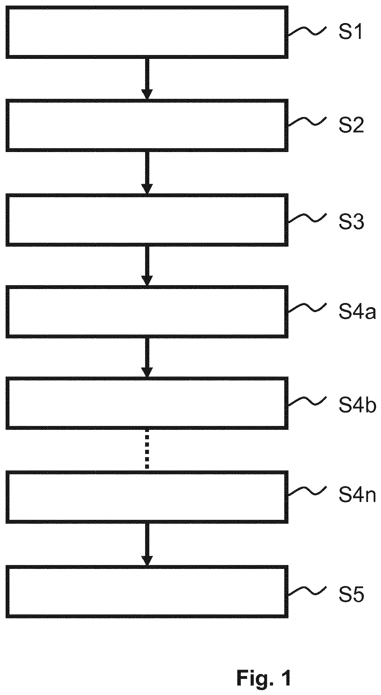

Fig. 1 zeigt ein Flussdiagramm zum Ablauf eines Ausführungsbeispiels eines erfindungsgemäßen Verfahrens zum Optimieren eines optischen Hilfsmittels mittels automatischer Bestimmung subjektiver Sehleistung. Das optische Hilfsmittel wird dabei spezifisch für ein Auge einer Person optimiert. Das optische Hilfsmittel ist beispielsweise ein Brillenglas.

In einem Schritt S1 wird ein Trainingsdatensatz bereitgestellt. Der bereitgestellte Trainingsdatensatz spiegelt die subjektive Sehleistung einer Vielzahl von realen Probanden beim Erkennen von Stimulusbildern wider.

Den realen Probanden wird beispielsweise jeweils mehrmals ein Landoltring (oder "Landolt-C") als Stimulusbild auf einem Monitor präsentiert. Die Präsentation wird jeweils unter standardisierten Bedingungen durchgeführt, insbesondere ist der Abstand zwischen Monitor und Proband festgelegt. Die Lücke des Landoltrings kann dabei eine Ausrichtung aus acht möglichen Winkeln aufweisen, nämlich "0°", "45°", "90°", "135°", "180°", "225°", "270°" oder "315°". Bei jeder Präsentation des Landoltrings wird zufällig eine dieser Lückenausrichtungen ausgewählt. Zusätzlich weist der Landoltring bei jeder Präsentation eine unterschiedliche Parametrisierung auf. Beispielsweise wird der Landoltring nach und nach mit unterschiedlicher Bildgröße präsentiert. Der Bildgröße entspricht angesichts des festgelegten Abstands zwischen Monitor und Proband dabei ein Sehwinkel. Der reale Proband hat zu jeder ihm präsentierten Parametrisierung (das heißt hier also Bildgröße bzw. Sehwinkel) des Landoltrings die von ihm subjektiv erkannte Lückenausrichtungen mitzuteilen. Diese subjektive Antwort des realen Probanden kann durch ein Betätigen verschiedener Tasten erfolgen. Dabei hat sich der reale Proband zwingend für eine konkrete Lückenausrichtung aus den möglichen Winkeln "0°", "45°", "90°", "135°", "180°", "225°", "270°" oder "315°" zu entscheiden. Für den Fall, dass der reale Proband keine Lückenausrichtung erkennen kann, hat er also zu raten.

Zum Erstellen des Trainingsdatensatzes werden beispielsweise zu jeder Lückenausrichtung 2.500 Präsentationen eines Landoltrings mit jeweils unterschiedlicher Bildgröße bei der Vielzahl von Probanden insgesamt durchgeführt. Die Anzahl der Präsentationen ist angesichts der acht verschiedenen Lückenausrichtungen des Landoltrings mit acht zu multiplizieren, liegt also beispielsweise bei 20.000.

Weiterhin werden bei jedem einzelnen realen Probanden mehrere Durchläufe durchgeführt. Dabei weisen die Stimulusbilder (das heißt die unterschiedlich großen und unterschiedlich ausgerichteten Landoltringe) in jedem Durchlauf eine unterschiedlich ausgeprägte optische Aberration, beispielsweise eine unterschiedliche Schärfe auf. Beispielsweise kann der Landoltring in sieben Durchläufen mit jeweils einer bestimmten Schärfe, das heißt mehr oder weniger scharf, präsentiert werden, wobei sich die Schärfe von Durchlauf zu Durchlauf unterscheidet. Entsprechend resultiert beispielsweise eine Gesamtzahl von sieben mal 20.000, also 140.000 Präsentationen für die Erstellung eines Trainingsdatensatzes.

Vorzugsweise umfassen die bereitgestellten Trainingsdatensätze die als Stimulusbilder präsentierten Landoltringe, die jeweilige tatsächliche Lückenausrichtung der präsentierten Landoltringe und die jeweilige subjektive Antwort der Probanden zur Lückenausrichtung der präsentierten Landoltringe.

In einem Schritt S2 wird ein künstliches neuronales Netzwerk mittels des Trainingsdatensatzes trainiert. Das künstliche neuronale Netzwerk ist beispielsweise ein Convolutional Neural Network (Abkürzung CNN), also ein neuronales Netzwerk, das eine Netzwerkarchitektur mit Faltungsneuronen aufweist. Die Netzwerkarchitektur kann verschiedene Anzahlen von Faltungsschichten aufweisen. Es können verschiedene Lernraten angewendet werden.

In einem Schritt S3 wird ein Stimulusbild generisch erstellt. Im hier vorgestellten Ausführungsbeispiel wird dabei der Landoltring mit acht verschiedenen Feature-Ausprägungen, nämlich mit seinen acht verschiedenen Lückenausrichtungen "0°", "45°", "90°", "135°", "180°", "225°", "270°" oder "315°", vorgesehen. Die verschiedenen Feature-Ausprägungen (Lückenausrichtungen) bilden dabei einen Feature-Raum.

In einem Schritt S4a wird die Schwelle der subjektiven Sehleistung für ein erstes optisches System bestimmt. Das erste optische System ist beispielsweise das Auge einer Person ohne optisches Hilfsmittel (oder mit einem optischen Hilfsmittel ohne optische Aberration). In einem Schritt S4b wird die Schwelle der subjektiven Sehleistung für ein zweites optisches System bestimmt. Das zweite optische System umfasst das Auge der Person und ein erstes optisches Hilfsmittel. Das erste optische Hilfsmittel ist vorzugsweise ein erstes Brillenglas, das heißt ein Brillenglas mit einem ersten Wert einer optischen Aberration, beispielsweise ein Brillenglas mit einem ersten Brechwert. Der Brechwert ist ein Beispiel für eine Eigenschaft des Brillenglases (optisches Hilfsmittel), die zum Hervorrufen einer zweiten Parametrisierung auf das Stimulusbild angewendet wird. Im hier betrachteten Beispiel wird als zweite Parametrisierung eine bestimmte Schärfe im Stimulusbild hervorgerufen. Die Schärfe resultiert aus einer Kombination der Brechwerte des Auges und des Brillenglases (optisches Hilfsmittel). Die im Stimulusbild resultierende Schärfe korrespondiert also zum Brechwert des gesamten optischen Systems. In einem Schritt S4n wird die Schwelle der subjektiven Sehleistung für ein n-tes optisches System bestimmt. Das n-te optische System umfasst das Auge der Person und ein (n-1)-tes optisches Hilfsmittel, beispielsweise ein Brillenglas mit einem (n-1)-ten Brechwert. Insgesamt wird also zu n optischen Systemen mit jeweils unterschiedlichem Brechwert für das Brillenglas die Schwelle der subjektiven Sehleistung bestimmt. Der Brechwert für das Brillenglas ist beim ersten optischen System im betrachteten Ausführungsbeispiel gleich 0.

In einem Schritt S5 wird das optische Hilfsmittel, also im genannten Beispiel das Brillenglas, ausgewählt, für das in den Schritten 4a bis 4n die höchste subjektive Sehleistung bestimmt wurde. Das dabei ausgewählte Brillenglas ist spezifisch für das Auge der Person optimiert. Im betrachteten Ausführungsbeispiel resultiert dabei das Stimulusbild mit der höchsten Schärfe aus der Kombination des Brechwerts des Auges mit dem Brechwert des optimierten Brillenglases.

Die "höchste subjektive Sehleistung" steht für die (je nach Parameterart) niedrigste bzw. höchste Lage der Schwelle der subjektiven Sehleistung. Im Fall der Bildgröße als erstem Parameter ergibt sich die höchste subjektive Sehleistung für das optische System aus den Schritten 4a bis 4n, zu dem die niedrigste Schwelle bestimmt wurde. Dieses optische System ermöglicht noch ein Erkennen des Stimulusbilds (Landoltrings) bei der verglichen mit den anderen in den Schritten 4a bis 4n untersuchten optischen Systemen kleinsten Bildgröße. Das in diesem optischen System vorgesehene optische Hilfsmittel (insbesondere Brillenglas) ist das also das für das Auge der Person optimierte optische Hilfsmittel (insbesondere Brillenglas). In Kombination mit diesem optimierten optischen Hilfsmittel (insbesondere Brillenglas) kann also das Auge der Person das höchste Auflösungsvermögen erzielen.

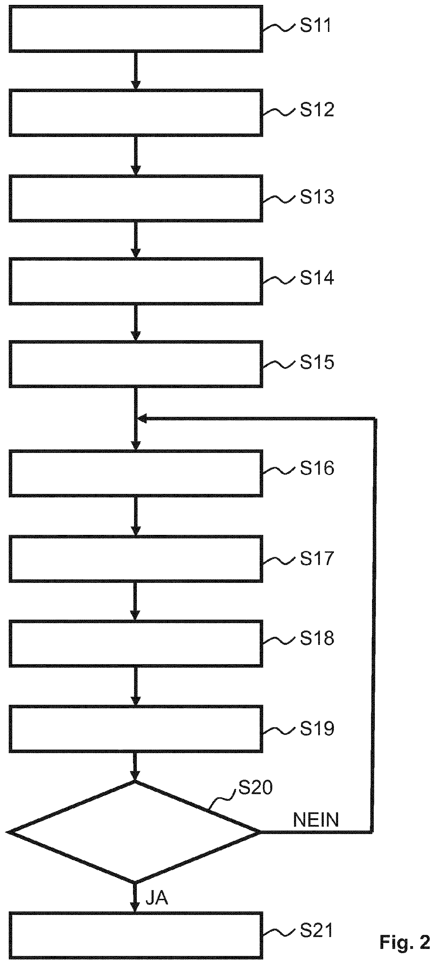

Fig. 2 zeigt ein das Ausführungsbeispiel der Fig. 1 ergänzendes Flussdiagramm zum Ablauf der jeweils in den Schritten 4a bis 4n vorgenommenen Bestimmung der Schwelle der subjektiven Sehleistung für das jeweils betrachtete optische System aus dem Auge der Person und dem jeweils unterschiedlich ausgestalteten optischen Hilfsmittel (insbesondere Brillenglas). Das Verfahren zur Bestimmung der Schwelle der subjektiven Sehleistung wird im Folgenden auch mit Schwellenbestimmungsverfahren bezeichnet. Das Schwellenbestimmungsverfahren wird durch eine Steuersoftware durchgeführt, die einen psychophysischen Algorithmus verwendet.

In einem Schritt S11 wird eine der möglichen Feature-Ausprägungen durch den psychophysischen Algorithmus ausgewählt. Dazu wird dem psychophysischen Algorithmus vorzugsweise der Feature-Raum zugeführt und durch den psychophysischen Algorithmus eine Feature-Ausprägung aus dem Feature-Raum mittels einer Zufallsverteilung ausgewählt. Je nach Beschaffenheit des Feature-Raums kann eine bestimmte Zufallsverteilung vorgesehen sein. Im hier betrachteten Beispiel eines Landoltrings wird also eine der acht möglichen Ausrichtungen der Lücke des Landoltrings ausgewählt. Es wird also ein Landoltring mit dieser Lückenausrichtung vorgesehen. Die Auswahl erfolgt zufällig, beispielsweise anhand einer Normalverteilung oder Gleichverteilung.

In einem Schritt S12 wird das Stimulusbild (Landoltring) durch den psychophysischen Algorithmus parametrisiert. Dazu wird dem psychophysischen Algorithmus vorzugsweise ein erster und ein zweiter Parameterraum zugeführt und durch den psychophysischen Algorithmus eine erste und eine zweite Parametrisierung vorgenommen. Die erste und zweite Parametrisierung betreffen verschiedene Parameter. Im hier betrachteten Ausführungsbeispiel wird die erste Parametrisierung des Stimulusbilds (Landoltring) anhand des ersten Parameters Bildgröße durchgeführt. Als erste Parametrisierung wird also ein konkreter Wert für die Bildgröße (im Folgenden auch einfach "eine Bildgröße" genannt) vorgesehen. Diese Bildgröße ist ein Anfangswert (Eingangswert) für den psychophysischen Algorithmus. Die Bildgröße entspricht einem bei sich aus ihr bei Standard-Bedingungen ergebenden Sehwinkel. Die zweite Parametrisierung resultiert im hier betrachteten Ausführungsbeispiel aus einer optischen Aberration, insbesondere aus einem sphärischen Defokus, des jeweiligen optischen Systems, das heißt des Auges der Person und/oder des Brillenglases. Als zweite Parametrisierung ergibt sich dabei eine konkrete Schärfe und/oder ein konkreter Kontrast im Stimulusbild. Über die Schritte 4a bis 4n hinweg wird die optische Aberration, insbesondere der sphärische Defokus, des zu optimierenden Brillenglases variiert. Dementsprechend variiert sich auch die zweite Parametrisierung über die Schritte 4a bis 4n hinweg. Vorzugsweise wird das Stimulusbild in jedem der Schritte 4a bis 4n mit einer anderen zweiten Parametrisierung, das heißt mit einem anderen Wert der Schärfe und/oder des Kontrasts vorgesehen.

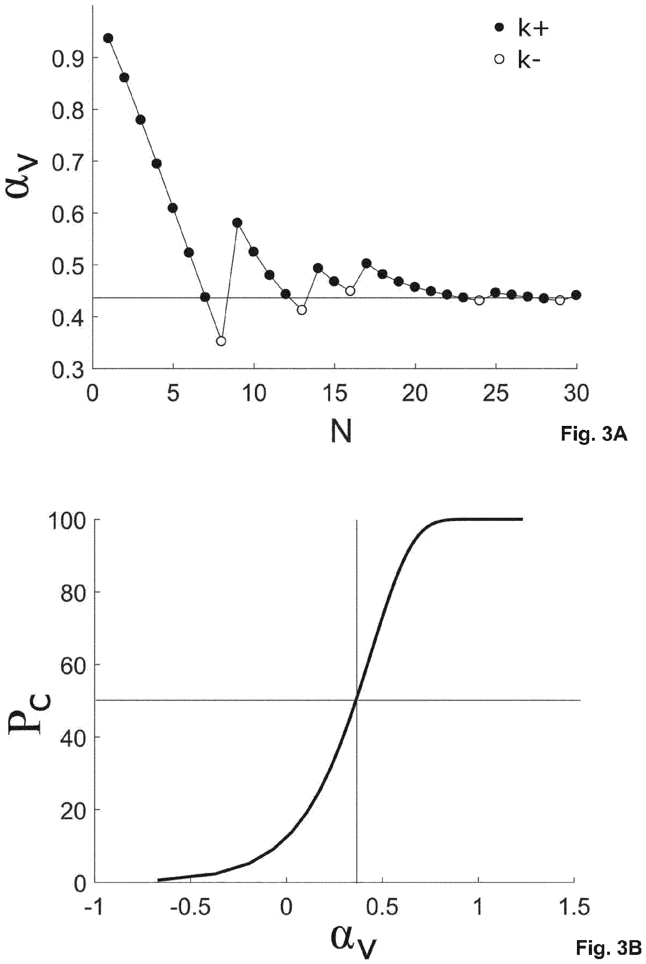

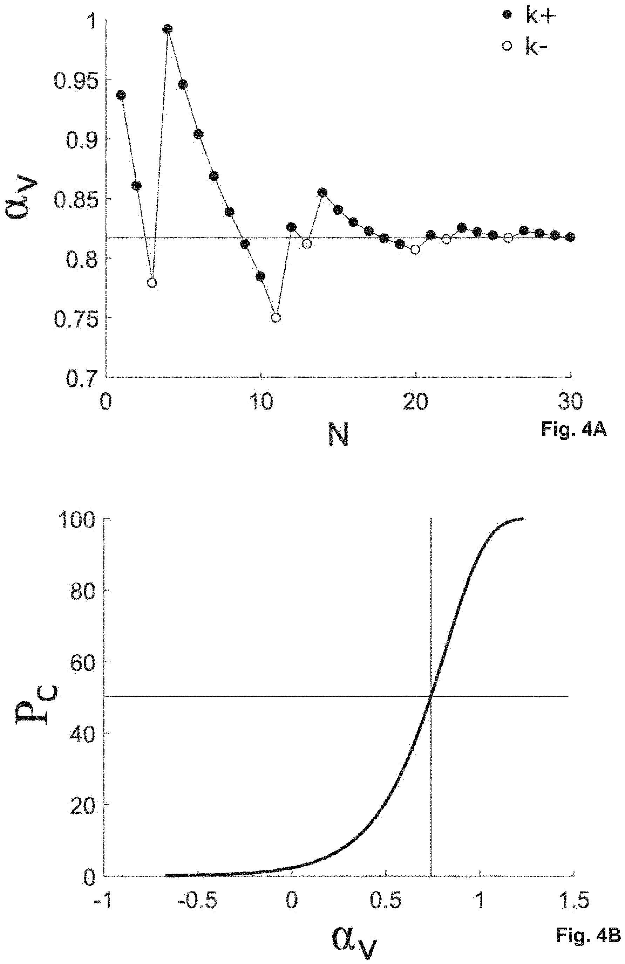

In einem Schritt S13 wird der psychophysische Algorithmus initialisiert. Der psychophysische Algorithmus hat das Ermitteln der Sehschwelle für das jeweils betrachtete optische System aus dem Auge der Person und/oder dem optischen Hilfsmittel (insbesondere Brillenglas) zum Ziel. Im hier betrachteten Ausführungsbeispiel bezieht sich die Sehschwelle auf eine minimale noch erkennbare Bildgröße bzw. dazu korrespondierend auf einen minimalen noch erkennbaren Sehwinkel. Der psychophysische Algorithmus geht dabei nach einem adaptiven Stufenverfahren, beispielsweise BestPEST, ein up-down-Staircase-Verfahren oder ein constant-stimuli-Verfahren, vor. Im hier betrachteten Ausführungsbeispiel wird nach BestPEST vorgegangen. Das Initialisieren des psychophysischen Algorithmus steht für das Beginnen des adaptiven Stufenverfahrens mit den im Schritt S12 ausgewählten Eingangswerten für die erste und zweite Parametrisierung. Im Schritt S13 wird weiterhin eine Erkennungswahrscheinlichkeitsfunktion bereitgestellt. Die Erkennungswahrscheinlichkeitsfunktion ordnet jeder ersten Parametrisierung, das heißt jeder Bildgröße, eine Erkennungswahrscheinlichkeit Pc zu. Bei der Erkennungswahrscheinlichkeitsfunktion handelt es sich um eine Sigmoidfunktion. Beispiele für Erkennungswahrscheinlichkeitsfunktionen sind in den Figuren 3B und 4B gezeigt.

In einem Schritt S14 klassifiziert das trainierte neuronale Netzwerk das Stimulusbilds (den Landoltring). Unter Klassifizieren des Stimulusbilds versteht man die Zuordnung des Stimulusbilds zu verschiedenen Kategorien, im hier betrachteten Fall des Landoltrings in die Kategorien "0°", "45°", "90°", "135°", "180°", "225°", "270°" oder "315°" sein. Die durch das trainierte neuronale Netzwerk erfolgte Zuordnung wird mit der tatsächlich vorhandenen Ausrichtung der Lücke des Landoltrings verglichen. Das neuronale Netzwerk stellt eine Klassifizierung des Stimulusbilds (Landoltrings) bereit. Bei korrekter Bestimmung der Ausrichtung der Lücke des Landoltrings durch das neuronale Netzwerk bestimmt der psychophysische Algorithmus die Klassifizierung als positiv. Bei falscher Bestimmung der Ausrichtung der Lücke des Landoltrings durch das neuronale Netzwerk bestimmt der psychophysische Algorithmus die Klassifizierung als negativ.

In einem Schritt S15 bestimmt der psychophysische Algorithmus eine Erkennungswahrscheinlichkeit Pc als Wert der Erkennungswahrscheinlichkeitsfunktion für die im Schritt S12 vorgesehene Bildgröße aufgrund der Klassifizierung aus dem Schritt S13. Bei positiver Klassifizierung bestimmt die Steuersoftware (der psychophysische Algorithmus) die Erkennungswahrscheinlichkeit Pc für die betreffende Bildgröße (erste Parametrisierung) als 100%. Bei negativer Klassifizierung bestimmt die Steuersoftware (der psychophysische Algorithmus) die Erkennungswahrscheinlichkeit Pc für die betreffende Bildgröße (erste Parametrisierung) als 0%. Die Erkennungswahrscheinlichkeitsfunktion kann bei BestPEST durch ein Anfitten an die zur betreffenden Bildgröße bestimmte Erkennungswahrscheinlichkeit Pc angepasst werden. Die Erkennungswahrscheinlichkeitsfunktion ist vorzugsweise eine gefittete Sigmoidfunktion.

In einem Schritt S16 wird erneut eine der möglichen Feature-Ausprägungen durch den psychophysischen Algorithmus ausgewählt. Die Auswahl erfolgt wiederum zufällig, beispielsweise anhand einer Normalverteilung oder Gleichverteilung. Im hier betrachteten Beispiel eines Landoltrings wird also erneut eine der acht möglichen Ausrichtungen der Lücke des Landoltrings ausgewählt und ein Landoltring mit dieser neu ausgewählten Lückenausrichtung im Stimulusbild vorgesehen. Es könnte zufälligerweise (mit einer Wahrscheinlichkeit von 1/8) weiterhin ein Landoltring mit unveränderter Lückenausrichtung vorgesehen werden. Alternativ dazu kann auch vorgesehen sein, dass solange zufällig eine der acht möglichen Ausrichtungen der Lücke des Landoltrings ausgewählt wird, bis eine im Vergleich zur zuvor ausgewählten Lückenausrichtung geänderte Lückenausrichtung ausgewählt ist.

In einem Schritt S17 passt der psychophysische Algorithmus die erste Parametrisierung des Stimulusbilds (also die Bildgröße des Landoltrings) auf der Grundlage der durch die Erkennungswahrscheinlichkeitsfunktion zugeordneten Erkennungswahrscheinlichkeit Pc an. Vorzugsweise wird die Bildgröße bei einer Erkennungswahrscheinlichkeit von über 50% (bzw. von zumindest 50%) derart angepasst, dass ein Erkennen der Lückenausrichtung des Landoltrings mit der neuen Bildgröße schwieriger wird, also die Bildgröße verringert wird. Entsprechend kann die Bildgröße bei einer Erkennungswahrscheinlichkeit von unter 50% derart angepasst werden, dass ein Erkennen der Lückenausrichtung des Landoltrings mit der neuen Bildgröße einfacher wird, also die Bildgröße erhöht wird. Weiterhin ist auch der Betrag der Differenz zwischen der neuen Bildgröße und der vorhergehenden Bildgröße im hier betrachteten Ausführungsbeispiel (BestPEST) von der durch die Erkennungswahrscheinlichkeitsfunktion zugeordneten Erkennungswahrscheinlichkeit abhängig. Vorzugsweise ist der Betrag dieser Differenz umso kleiner, je näher die zugeordnete Erkennungswahrscheinlichkeit bei 50% liegt. Das Ziel des Anpassens der Bildgröße besteht darin, die Bildgröße der Sehschwelle anzunähern.

Je nach Art des vorzugsweise verwendeten psychophysischen Algorithmus kann vorgesehen sein, dass beim Anpassen der Bildgröße (erste Parametrisierung) im Schritt S17 immer eine Bildgröße eingestellt wird, die zuvor noch nicht eingestellt war (z.B. BestPEST), oder dass eine bereits zuvor verwendete Bildgröße im Schritt S17 erneut eingestellt werden darf (z.B. up-down-Staircase-Verfahren, constant-stimuli-Verfahren). Entsprechend kann eine bestimmte erste Parametrisierung je nach Art des psychophysischen Algorithmus nur einmal oder mehrmals abgefragt werden.

In einem Schritt S18 klassifiziert das trainierte neuronale Netzwerk das angepasste Stimulusbild (Landoltring). Die Vorgehensweise erfolgt dabei wie in Schritt S14. Das neuronale Netzwerk stellt erneut eine Klassifizierung des Stimulusbilds (Landoltrings) bereit. Bei korrekter Bestimmung der Ausrichtung der Lücke des Landoltrings ist die Klassifizierung positiv. Bei falscher Bestimmung der Ausrichtung der Lücke des Landoltrings ist die Klassifizierung negativ.

In einem Schritt S19 bestimmt der psychophysische Algorithmus die Erkennungswahrscheinlichkeit Pc als Wert der Erkennungswahrscheinlichkeitsfunktion für die im Schritt S17 angepasste Bildgröße aufgrund der Klassifizierung aus dem Schritt S18. Dabei bestimmt der psychophysische Algorithmus die Erkennungswahrscheinlichkeit Pc für die angepasste Bildgröße bei positiver Klassifizierung als 100% und bei negativer Klassifizierung als 0%. Im hier betrachteten Ausführungsbeispiel (BestPEST) wird die Bildgröße in sehr feinen Abstufungen angepasst. Daher kommt es normalerweise bei BestPEST nicht zum wiederholten Abfragen einer bestimmten Bildgröße. Bei anderen adaptiven Stufenverfahren oder ausnahmsweise bei BestPEST kann bei positiver Klassifizierung, sofern die betreffende Bildgröße bereits abgefragt wurde, ein Mittelwert aus 100% und der bereits zuvor (in einem vorangehenden Durchgang beim Schritt S19 oder bereits beim Schritt S15) zur gleichen Bildgröße ermittelten Erkennungswahrscheinlichkeit durch den psychophysischen Algorithmus bestimmt werden. Bei negativer Klassifizierung kann, sofern die betreffende Bildgröße bereits abgefragt wurde, der psychophysische Algorithmus die Erkennungswahrscheinlichkeit Pc für die angepasste Bildgröße als einen Mittelwert aus 0% und der bereits zuvor (in einem vorangehenden Durchgang beim Schritt S19 oder bereits beim Schritt S15) zur gleichen Bildgröße ermittelten Erkennungswahrscheinlichkeit bestimmen. Die Mittelung kann dann jeweils derart erfolgen, dass jede Abfrage der betreffenden Bildgröße die gleiche Gewichtung erhält. Bei BestPEST sind vorzugsweise bei der Abfrage einer bestimmten Bildgröße (erste Parametrisierung) nur die Erkennungswahrscheinlichkeiten Pc gleich 0% oder 100% möglich. Die Erkennungswahrscheinlichkeitsfunktion kann bei BestPEST durch ein Anfitten an die zur angepassten Bildgröße bestimmte Erkennungswahrscheinlichkeit angepasst werden. Die Erkennungswahrscheinlichkeitsfunktion ist vorzugsweise eine an die zur angepassten Bildgröße bestimmte Erkennungswahrscheinlichkeit sowie an in vorangehenden Durchgängen beim Schritt S19 bzw. beim Schritt S15 zu den jeweiligen Bildgrößen bestimmte Erkennungswahrscheinlichkeiten angefittete Sigmoidfunktion (siehe Fig. 3B und 4B).

In einem Schritt S20 überprüft der psychophysische Algorithmus, ob ein Abbruchkriterium erreicht ist. Falls ein Abbruchkriterium erreicht ist, folgt ein Schritt S21 (siehe unten). Falls kein Abbruchkriterium erreicht ist, werden erneut die Schritte S16 bis S20 wiederholt. Dabei sieht der psychophysische Algorithmus bei jedem Durchlaufen des Schritts S16 eine beliebige Lückenausrichtung des Landoltrings vor. Der psychophysische Algorithmus führt solange eine Rekursion durch, bis die Schwelle der subjektiven Sehleistung aufgefunden ist, das heißt bis die erste Parametrisierung (Bildgröße) sich hinreichend der Schwelle genähert hat und/oder die Schwelle erreicht hat, so dass nachfolgend (im Schritt S21) die Schwelle bestimmt werden kann.

Im hier betrachteten Ausführungsbeispiel wird das Abbruchkriterium durch einen der folgenden Fälle erreicht:
- Erreichen einer vorbestimmten Anzahl von Wiederholungen des Durchlaufens der Schritte S16 bis S19. Dazu kann ein Zähler vorgesehen sein, der sich beispielsweise im Schritt S16 um 1 erhöht.
- Erreichen einer vorbestimmten Anzahl von Unterschreitungen eines vorbestimmten Grenzwerts durch eine Differenz von beim wiederholten Durchlaufen des Schritts S17 aufeinanderfolgenden angepassten ersten Parametrisierungen (Bildgrößen). Dabei kann die vorbestimmte Anzahl beispielsweise gleich drei sein, so dass ein dreimaliges Unterschreiten zur Erfüllung des Abbruchkriteriums erforderlich ist. Dieses Kriterium beschreibt also eine (hinreichende) Konvergenz der ersten Parametrisierung (Bildgröße) mit der Schwelle der subjektiven Sehleistung. Im betrachteten Ausführungsbeispiel wird die Differenz aus in aufeinanderfolgenden Durchläufen des Schritts S17 angepassten Bildgrößen bzw. den angepassten Bildgrößen entsprechenden Sehwinkeln (Δαᵥ) gebildet. Maßgeblich ist dabei der Betrag der Differenz. Es ist also gleichgültig, ob die Konvergenz zu größeren Werten der Bildgröße bzw. des Sehwinkels (Δαᵥ) hin oder zu kleineren Werten der Bildgröße bzw. des Sehwinkels (Δαᵥ) hin erfolgt.
- Erreichen einer vorbestimmten Anzahl von Reversals. "Reversals" stehen für die in den Fig. 3A und 4A gezeigten Umkehrpunkte (bzw. lokalen Extrema) in einer Abfolge der gemäß Schritt S17 angepassten Bildgröße beim Wiederholen der Schritte S16 bis S20. Ein "Reversal" kann also auch definiert werden als ein Wechsel zwischen positiver und negativer Klassifizierung (siehe Schritt S14 bzw. S18) in der Abfolge der Fig. 3A bzw. 4A. Ein "Reversal" ist also ein Hin- und Herspringen zwischen positiver und negativer Klassifizierung bzw. zwischen negativer und positiver Klassifizierung. Das Erreichen einer hinreichend hohen Anzahl (also insbesondere der vorbestimmten Anzahl) von Reversals ist ein Anzeichen dafür, dass die erste Parametrisierung (Bildgröße) genau um den Wert der Schwelle pendelt. Das heißt, dass die Schwelle wertmäßig genau zwischen den an den Reversals erreichten Bildgrößen liegt.

Im Schritt S21 bestimmt der psychophysische Algorithmus die Schwelle der subjektiven Sehleistung auf der Grundlage der zu den jeweiligen ersten Parametrisierungen (Bildgrößen) des Stimulusbilds bestimmten Erkennungswahrscheinlichkeiten. Je nach Art des eingesetztem adaptiven Stufenverfahren kann sich die Vorgehensweise beim Bestimmen der Schwelle der subjektiven Sehleistung unterscheiden. Im Fall einer vorbestimmten Anzahl von Wiederholung der Schritte S16 bis S19 kann bei BestPEST beispielsweise die bei der letzten Durchführung des Schritts S17 erhaltene angepasste Bildgröße (erste Parametrisierung) als Schwelle übernommen werden. Alternativ dazu kann bei BestPEST auch die Bildgröße (erste Parametrisierung), für die die gefittete Erkennungswahrscheinlichkeitsfunktion die Erkennungswahrscheinlichkeit 50% ergibt, als Schwelle bestimmt werden.

Fig. 3A zeigt ein Diagramm zum Ablauf eines Schwellenbestimmungsverfahrens zu einem ersten optischen Hilfsmittel. Das erste optische Hilfsmittel ist im hier betrachteten Ausführungsbeispiel ein erstes Brillenglas bzw. ein Brillenglas mit einem ersten Brechwert. Fig. 4A zeigt ein Diagramm zum Ablauf eines Schwellenbestimmungsverfahrens zu einem zweiten optischen Hilfsmittel. Das zweite optische Hilfsmittel ist im hier betrachteten Ausführungsbeispiel ein zweites Brillenglas bzw. ein Brillenglas mit einem zweiten Brechwert. Der Begriff "Diagramm zum Ablauf eines Schwellenbestimmungsverfahrens" wird im Folgenden verkürzt mit Schwellenbestimmungsdiagramm bezeichnet. In den beiden Schwellenbestimmungsdiagrammen ist nach rechts jeweils die fortlaufende Zahl der Durchgänge N des Schwellenbestimmungsverfahrens aufgetragen. Der erste Durchgang (N = 1) bezieht sich dabei auf das Durchlaufen der Schritte S11 bis S15, die folgenden Durchgänge jeweils auf das Durchlaufen der Schritte S16 bis S20 gemäß Fig. 2. Die beiden Schwellenbestimmungsdiagramme zeigen jeweils eine graphische Auftragung der ersten Parametrisierung über die fortlaufende Zahl der Durchgänge N. Im hier betrachteten Ausführungsbeispiel handelt es sich bei der ersten Parametrisierung um die Bildgröße bzw. der dazu korrespondierende Sehwinkel αᵥ. Zu jedem Durchgang N ist der abgefragte Sehwinkel αᵥ eingezeichnet. Dabei ist der Sehwinkel αᵥ für den Fall einer positiven Klassifizierung k+ durch das trainierte neuronale Netzwerk als ausgefüllte Kreisscheibe eingezeichnet und für den Fall einer negativen Klassifizierung k-durch das trainierte neuronale Netzwerk als Kreisring eingezeichnet.

Fig. 3B zeigt eine Erkennungswahrscheinlichkeitsfunktion zum ersten optischen Hilfsmittel, das heißt zum ersten Brillenglas bzw. zum Brillenglas mit erstem Brechwert. Fig. 4B zeigt eine Erkennungswahrscheinlichkeitsfunktion zum zweiten optischen Hilfsmittel, das heißt zum zweiten Brillenglas bzw. zum Brillenglas mit zweitem Brechwert. Dabei ist jeweils eine Erkennungswahrscheinlichkeit Pc über dem Sehwinkel αᵥ aufgetragen. Für jeden Sehwinkel αᵥ bestimmt dazu der psychophysische Algorithmus durch das Anfitten einer Sigmoidfunktion an die im Schritt S15 zur Eingangs-Bildgröße und jeweils im Schritt S19 zu den jeweiligen angepassten Bildgrößen bestimmten Erkennungswahrscheinlichkeiten eine Erkennungswahrscheinlichkeit Pc. Da die im Schritt S15 zur Eingangs-Bildgröße und jeweils im Schritt S19 zu den jeweiligen angepassten Bildgrößen bestimmten Erkennungswahrscheinlichkeiten im Fall von BestPEST lediglich die Werte 0% oder 100% aufweisen, wurde auf eine Darstellung dieser Erkennungswahrscheinlichkeiten in den Fig. 3B und 4B verzichtet.

Im hier betrachteten Ausführungsbeispiel wird als adaptives Stufenverfahren BestPEST eingesetzt. Ordnet die Erkennungswahrscheinlichkeitsfunktion einem Sehwinkel αᵥ eine Erkennungswahrscheinlichkeit Pc von zumindest 50% zu, wird der Sehwinkel αᵥ im nachfolgenden Durchgang (N + 1) bei der Anpassung im Schritt S17 reduziert. Der Betrag der Reduktion des Sehwinkels, also der Betrag der Sehwinkeldifferenz Δαᵥ, hängt dabei auch von der zugeordneten Erkennungswahrscheinlichkeit Pc ab. Er ist vorzugsweise umso geringer, je näher die Erkennungswahrscheinlichkeit Pc bei 50% liegt. Ordnet die Erkennungswahrscheinlichkeitsfunktion einem Sehwinkel αᵥ eine Erkennungswahrscheinlichkeit Pc von unter 50% zu, wird der Sehwinkel αᵥ im nachfolgenden Durchgang (N + 1) bei der Anpassung im Schritt S17 erhöht.

Der Betrag der Erhöhung des Sehwinkels, also der Betrag der Sehwinkeldifferenz Δαᵥ, hängt auch hier von der durch die Erkennungswahrscheinlichkeitsfunktion zugeordneten Erkennungswahrscheinlichkeit Pc ab. Er ist vorzugsweise umso geringer, je näher die Erkennungswahrscheinlichkeit Pc bei 50% liegt. Nach dem Erreichen einer vorbestimmten Anzahl von Durchgängen N, im hier betrachteten Ausführungsbeispiel N = 30, wird die Abfrage weiterer Sehwinkel αᵥ abgebrochen.

Der Sehwinkel αᵥ, für den eine Erkennungswahrscheinlichkeit Pc von 50% erzielt wird, wird als Wert der Schwelle bestimmt. Der Wert der Schwelle ergibt sich also graphisch in den Fig. 3B und 4B durch eine Projektion der 50%-Linie mittels einer jeweils eingezeichneten vertikalen Linie auf die Rechtsachse. Die derart bestimmte Schwelle ist in Fig. 3A und Fig. 4A als horizontale Linie eingezeichnet.

Im hier betrachteten Ausführungsbeispiel ergibt sich für das Brillenglas mit dem ersten Brechwert (Fig. 3A und 3B) ein niedrigerer Wert für die Schwelle als für das Brillenglas mit dem zweiten Brechwert (Fig. 4A und 4B). Das Brillenglas mit dem ersten Brechwert erlaubt dem Auge der Person also noch ein Erkennen von Bildgrößen (bzw. Sehwinkeln), die nicht mehr mit dem Brillenglas mit dem zweiten Brechwert erkannt werden können. Das Brillenglas mit dem ersten Brechwert bietet also spezifisch für die Person das höhere Auflösungsvermögen. Sofern nur diese beiden Brillengläser dem Optimierungsverfahren (siehe Fig. 1) unterzogen wurden, wird also das Brillenglas mit dem ersten Brechwert im Schritt S5 des Optimierungsverfahrens als das für das Auge der Person optimierte Brillenglas ausgewählt.

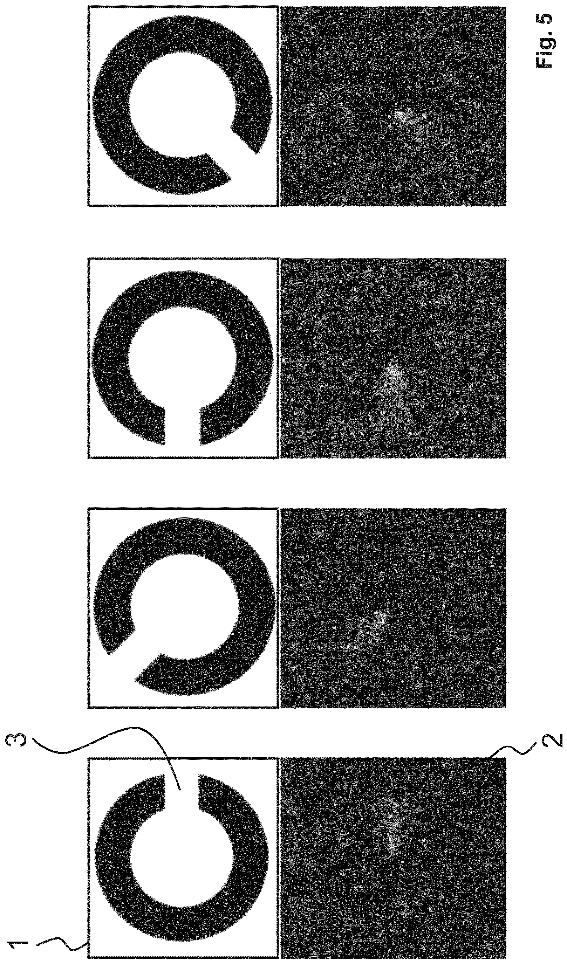

Fig. 5 zeigt eine Gegenüberstellung von Stimulusbildern 1 und jeweils zugehöriger Visualisierung 2 erlernter Gewichte eines trainierten neuronalen Netzwerks. Fig. 5 zeigt also das Ergebnis des Trainierens des neuronalen Netzwerks. Das neuronale Netzwerk ist in diesem Beispiel ein Convolutional Neural Network. Bei den Stimulusbildern 1 handelt es sich jeweils um einen Landoltring mit gleicher Bildgröße (erste Parametrisierung) und gleicher Schärfe (zweite Parametrisierung). Die gezeigten Stimulusbilder unterscheiden sich in der Ausrichtung der Lücke 3 des Landoltrings, das heißt in der Feature-Ausprägung. Beim ersten gezeigten Landoltring ist die Lücke 3 bei 0°, beim zweiten gezeigten Landoltring bei 135°, beim dritten gezeigten Landoltring bei 180° und beim vierten gezeigten Landoltring bei 225°. Auf eine Berücksichtigung der weiteren möglichen Feature-Ausprägungen des Landoltrings (Lückenausrichtungen 45°, 90°, 270° und 315°) wurde zwecks der besseren Übersichtlichkeit in Fig. 5 verzichtet. Die Visualisierung 2 zur Lückenausrichtung 0° zeigt beispielsweise eine Darstellung aller Gewichte des trainierten neuronalen Netzwerks, die der Lückenausrichtung 0° entsprechen. Auch zu den weiteren Feature-Ausprägungen (Lückenausrichtungen) sind jeweils die Darstellungen der diesen Feature-Ausprägungen jeweils entsprechenden Gewichte des trainierten neuronalen Netzwerks als Visualisierung 2 gezeigt. Die Stimulusbilder 1 und die zugehörigen Visualisierungen 2 weisen im gezeigten Beispiel jeweils 500x500 Pixel auf.

### Bezugszeichenliste

- 1: Stimulusbilder
- 2: Visualisierung
- 3: Lücke

- αᵥ: Sehwinkel
- Δαᵥ: Sehwinkeldifferenz
- k+: positive Klassifizierung
- k-: negative Klassifizierung
- N: Durchgang
- Pc: Erkennungswahrscheinlichkeit

## Patentansprüche

1. Verfahren zum Optimieren eines optischen Hilfsmittels mittels automatischer Bestimmung subjektiver Sehleistung unter Verwendung von maschinellem Lernen umfassend die folgenden Schritte:
a) Bereitstellen zumindest eines Trainingsdatensatzes,
b) Trainieren eines künstlichen neuronalen Netzwerks mittels des zumindest einen Trainingsdatensatzes,
c) Generisches Erstellen eines Stimulusbilds,
d) Parametrisieren des Stimulusbilds, wobei eine erste und eine zweite Parametrisierung vorgenommen wird, und wobei vorzugsweise die erste Parametrisierung des Stimulusbilds zumindest durch eine Bildgröße des Stimulusbilds, einen Stimuluskontrast und/oder eine Stimulusform erfolgt,
e) Bereitstellen einer Erkennungswahrscheinlichkeitsfunktion,
f) Klassifizieren des Stimulusbilds durch das trainierte neuronale Netzwerk zum Bereitstellen einer Klassifizierung des Stimulusbilds,
g) Bestimmen einer Erkennungswahrscheinlichkeit für die im Schritt d) vorgenommene erste Parametrisierung auf der Grundlage der Klassifizierung des Stimulusbilds,
h) Anpassen der ersten Parametrisierung des Stimulusbilds auf der Grundlage der bestimmten Erkennungswahrscheinlichkeit,
i) Klassifizieren des angepassten Stimulusbilds durch das trainierte neuronale Netzwerk zum Bereitstellen einer Klassifizierung des angepassten Stimulusbilds,
j) Bestimmen der Erkennungswahrscheinlichkeit für die im Schritt h) angepasste erste Parametrisierung auf der Grundlage der Klassifizierung des angepassten Stimulusbilds,
k) Wiederholen der Schritte h) bis j) bis zum Erreichen eines Abbruchkriteriums in Bezug auf ein Auffinden einer Schwelle der subjektiven Sehleistung,
l) Bestimmen der Schwelle der subjektiven Sehleistung,
m) mehrfach wiederholtes Durchlaufen der Schritte d) bis l) mit jeweils unterschiedlicher zweiter Parametrisierung, wobei die zweite Parametrisierung des Stimulusbilds jeweils das Anwenden zumindest einer Eigenschaft des optischen Hilfsmittels auf das Stimulusbild umfasst,
n) Optimieren des optischen Hilfsmittels durch Auswählen der zweiten Parametrisierung mit der beim Schritt l) höchsten bestimmten subjektiven Sehleistung.

2. Verfahren nach Anspruch 1, wobei das Abbruchkriterium eines oder mehrere der folgenden Kriterien umfasst:
Erreichen einer vorbestimmten Anzahl von Wiederholungen des Durchlaufens der Schritte h) bis j) gemäß Schritt k),
Erreichen einer vorbestimmten Anzahl von Unterschreitungen eines vorbestimmten Grenzwerts durch eine Differenz von beim wiederholten Durchlaufen der Schritte h) bis j) gemäß Schritt k) aufeinanderfolgenden angepassten ersten Parametrisierungen,
Erreichen einer vorbestimmten Anzahl von Reversals.

3. Verfahren nach Anspruch 1 oder 2, wobei die beim Parametrisieren des Stimulusbilds nach Schritt d) vorgenommene zweite Parametrisierung das Anwenden zumindest einer Eigenschaft eines Auges einer Person, insbesondere einer optischen Aberration des Auges der Person, auf das Stimulusbild umfasst.

4. Verfahren nach einem der vorangehenden Ansprüche, wobei die nach Schritt m) beim Parametrisieren des Stimulusbilds nach Schritt d) auf das Stimulusbild angewandte Eigenschaft des optischen Hilfsmittels eine optische Aberration des optischen Hilfsmittels umfasst.

5. Verfahren nach Anspruch 4, wobei die optische Aberration des Auges der Person gemessen und/oder berechnet ist, und/oder wobei die optische Aberration des optischen Hilfsmittels gemessen und/oder berechnet ist.

6. Verfahren nach einem der vorangehenden Ansprüche, wobei der Schritt c) das Vorsehen eines mehrere Feature-Ausprägungen umfassenden Feature-Raums umfasst,
wobei im Schritt d) vor dem Parametrisieren des Stimulusbilds eine der Feature-Ausprägungen aus dem Feature-Raum ausgewählt und im Stimulusbild vorgesehen wird,
und wobei im Schritt h) vor dem Anpassen der ersten Parametrisierung erneut eine der Feature-Ausprägungen aus dem Feature-Raum ausgewählt und im Stimulusbild vorgesehen wird.

7. Verfahren nach einem der vorangehenden Ansprüche, wobei das neuronale Netzwerk ein Convolutional Neural Network ist.

8. Verfahren nach einem der vorangehenden Ansprüche, wobei die Schritte b), c), d), e), g), h), j), k), l), m) und/oder n) durch eine Steuersoftware durchgeführt werden, wobei die Steuersoftware vorzugsweise einen psychophysischen Algorithmus verwendet oder ein psychophysischer Algorithmus ist.

9. Verfahren nach Anspruch 8, wobei der psychophysische Algorithmus nach einem adaptiven Stufenverfahren vorgeht.

10. Verfahren zum Herstellen eines durch ein Verfahren nach einem der vorangehenden Ansprüche optimierten optischen Hilfsmittels, umfassend Bestimmen von Oberflächenbearbeitungsdaten gemäß der in Schritt n) ausgewählten zweiten Parametrisierung des optimierten optischen Hilfsmittels, Zuführen der Oberflächenbearbeitungsdaten an einen Automaten zur mechanischen Bearbeitung von optischen Oberflächen zum mechanischen Bearbeiten der optischen Oberflächen von Rohlingen gemäß den Oberflächenbearbeitungsdaten.

11. Verfahren nach Anspruch 10, wobei das mechanische Bearbeiten der optischen Oberflächen Schleifen und/oder Polieren umfasst, die Oberflächenbearbeitungsdaten Schleifdaten und/oder Polierdaten umfassen und der Automat zur mechanischen Bearbeitung von optischen Oberflächen einen Schleif- und/oder Polierautomaten umfasst.

12. Vorrichtung zum Herstellen von optimierten optischen Hilfsmitteln, umfassend
einen Automaten zur mechanischen Bearbeitung von optischen Oberflächen,
eine Einrichtung zum Bestimmen von Oberflächenbearbeitungsdaten gemäß der in Schritt n) ausgewählten zweiten Parametrisierung des optimierten optischen Hilfsmittels,
eine Auswerteeinrichtung zum Durchführen eines Verfahrens nach einem der Ansprüche 1 bis 9, umfassend Schnittstellen zum Einlesen von Trainingsdatensätzen und zur Eingabe und/oder Auswahl von Stimulusbildern sowie von Parametrisierungsdaten zu den ersten und zweiten Parametrisierungen.

13. Vorrichtung nach Anspruch 12, weiterhin umfassend eine Testeinrichtung zur Überprüfung der bearbeiteten optimierten optischen Hilfsmittel.

14. Computerprogramm mit auf einem Prozessor ablauffähigem Programmcode zum Ausführen der Verfahrensschritte eines Verfahrens nach einem der Ansprüche 1 bis 9.

15. Nichttransitorisches Speichermedium mit einem darauf gespeicherten Computerprogramm nach Anspruch 14.

## Claims

1. Method for optimizing an optical aid by automatically determining subjective visual performance by means of machine learning, comprising the following steps:
a) providing at least one training dataset,
b) training an artificial neural network by means of the at least one training dataset,
c) generically creating a stimulus image,
d) parameterizing the stimulus image, wherein a first and a second parameterization is performed, and wherein preferably the first parameterization of the stimulus image is implemented at least by way of an image size of the stimulus image, a stimulus contrast and/or a stimulus shape,
e) providing a detection probability function,
f) classifying the stimulus image by means of the trained neural network for providing a classification of the stimulus image,
g) determining a detection probability for the first parameterization, performed in step d), on the basis of the classification of the stimulus image,
h) adapting the first parameterization of the stimulus image on the basis of the determined detection probability,
i) classifying the adapted stimulus image by means of the trained neural network for providing a classification of the adapted stimulus image,
j) determining the detection probability for the first parameterization, adapted in step h), on the basis of the classification of the adapted stimulus image,
k) repeating steps h) to j) until reaching a termination criterion with respect to finding a threshold of the subjective visual performance,
l) determining the threshold of the subjective visual performance,
m) repeatedly running through steps d) to 1) multiple times with a different second parameterization in each case, wherein each second parameterization of the stimulus image comprises the application of at least one property of the optical aid to the stimulus image,
n) optimizing the optical aid by means of selecting the second parameterization with the highest subjective visual performance determined in step 1).

2. Method according to Claim 1, wherein the termination criterion comprises one or more of the following criteria:
reaching a predetermined number of repetitions of running through steps h) to j) according to step k),
reaching a predetermined number of times of undershooting a predetermined threshold value by a difference of consecutive adapted first parameterizations while repeatedly running through steps h) to j) according to step k),
reaching a predetermined number of reversals.

3. Method according to Claim 1 or 2, wherein the second parameterization, performed while parameterizing the stimulus image according to step d), comprises the application of at least one property of an eye of a person, in particular an optical aberration of the eye of the person, to the stimulus image.

4. Method according to any of the preceding claims, wherein the property of the optical aid, said property being applied to the stimulus image according to step m) while parameterizing the stimulus image according to step d), comprises an optical aberration of the optical aid.

5. Method according to Claim 4, wherein the optical aberration of the eye of the person is measured and/or calculated, and/or wherein the optical aberration of the optical aid is measured and/or calculated.

6. Method according to any of the preceding claims, wherein step c) comprises the provision of a feature space comprising a plurality of feature characteristics,
wherein in step d) before parameterizing the stimulus image, one of the feature characteristics is selected from the feature space and is provided in the stimulus image,
and wherein in step h) before adapting the first parameterization, once again one of the feature characteristics is selected from the feature space and is provided in the stimulus image.

7. Method according to any of the preceding claims, wherein the neural network is a convolutional neural network.

8. Method according to any of the preceding claims, wherein steps b), c), d), e), g), h), j), k), l), m) and/or n) are carried out by a control software, wherein the control software preferably uses a psychophysical algorithm or is a psychophysical algorithm.

9. Method according to Claim 8, wherein the psychophysical algorithm proceeds according to an adaptive step method.

10. Method for manufacturing an optical aid, optimized by means of a method according to any of the preceding claims, comprising
determining surface processing data according to the second parameterization, selected in step n), of the optimized optical aid,
supplying the surface processing data to a machine for mechanical processing of optical surfaces for mechanical processing of the optical surfaces of blanks according to the surface processing data.

11. Method according to Claim 10, wherein the mechanical processing of the optical surfaces comprises grinding and/or polishing, the surface processing data comprise grinding data and/or polishing data and the machine for mechanical processing of optical surfaces comprises a grinding and/or polishing machine.

12. Device for manufacturing optimized optical aids, comprising
a machine for mechanical processing of optical surfaces,
an apparatus for determining surface processing data according to the second parameterization, selected in step n), of the optimized optical aid,
an evaluation apparatus for carrying out a method according to any of Claims 1 to 9, comprising interfaces for reading in training datasets and for inputting and/or selecting stimulus images and parameterization data for the first and second parameterizations.

13. Device according to Claim 12, furthermore comprising a test apparatus for checking the processed optimized optical aid.

14. Computer program with program code, executable on a processor, for implementing the method steps of a method according to any of Claims 1 to 9.

15. Non-transitory storage medium with a computer program according to Claim 14 stored thereon.

## Revendications

1. Procédé d'optimisation d'un moyen optique par détermination automatique des capacités visuelles subjectives par le biais d'un apprentissage machine, comprenant les étapes suivante :
a) fourniture d'au moins un ensemble de données d'apprentissage,
b) apprentissage d'un réseau neuronal artificiel au moyen d'au moins un ensemble de données d'apprentissage,
c) création générique d'une image de stimulus,
d) paramétrage de l'image de stimulus, dans lequel un premier et un deuxième paramétrage sont effectués, et dans lequel le premier paramétrage de l'image de stimulus est de préférence effectué au moins par le biais d'une taille d'image de l'image de stimulus, d'un contraste de stimulus et/ou d'une forme de stimulus,
e) fourniture d'une fonction de probabilité de détection,
f) classification de l'image de stimulus par le réseau neuronal soumis à l'apprentissage pour fournir une classification de l'image de stimulus,
g) détermination d'une probabilité de détection pour le premier paramétrage effectué à l'étape d) sur la base de la classification de l'image de stimulus,
h) ajustement du premier paramétrage de l'image de stimulus sur la base de la probabilité de détection déterminée,
i) classification de l'image de stimulus ajustée par le réseau neuronal soumis à l'apprentissage afin de fournir une classification de l'image de stimulus ajustée,
j) détermination de la probabilité de détection pour le premier paramétrage ajusté à l'étape h) sur la base de la classification de l'image de stimulus ajustée,
k) répétition des étapes h) à j) jusqu'à ce qu'un critère d'interruption soit atteint en ce qui concerne l'obtention d'un seuil de capacité visuelle subjective,
l) détermination du seuil de capacité visuelle subjective,
m) exécution répétée plusieurs fois des étapes d) à 1) avec à chaque fois un second paramétrage différent, dans lequel le second paramétrage de l'image de stimulus comprend respectivement l'application d'au moins une propriété du moyen optique à l'image de stimulus,
n) optimisation du moyen optique par sélection du second paramétrage présentant la plus haute capacité visuelle subjective déterminée à l'étape 1).

2. Procédé selon la revendication 1, dans lequel le critère d'interruption comprend un ou plusieurs des critères suivants :
atteindre un nombre prédéterminé de répétitions du passage par les étapes h) à j) conformément à l'étape k),
atteindre un nombre prédéterminé de fois où une différence entre les premiers paramétrages successifs ajustés lors de l'exécution répétée des étapes h) à j) conformément à l'étape k) est inférieur à une valeur limite prédéterminée,
atteindre un nombre prédéterminé d'inversions.

3. Procédé selon la revendication 1 ou 2, dans lequel le second paramétrage effectué lors du paramétrage de l'image de stimulus conformément à l'étape d) comprend l'application d'au moins une propriété d'un œil d'une personne, en particulier une aberration optique de l'œil de la personne, à l'image de stimulus.

4. Procédé selon l'une des revendications précédentes, dans lequel la propriété du moyen optique appliquée à l'image de stimulus conformément à l'étape m) lors du paramétrage de l'image de stimulus conformément à l'étape d) comprend une aberration optique du moyen optique.

5. Procédé selon la revendication 4, dans lequel l'aberration optique de l'œil de la personne est mesurée et/ou calculée, et/ou dans lequel l'aberration optique du moyen optique est mesurée et/ou calculée.

6. Procédé selon l'une des revendications précédentes, dans lequel l'étape c) comprend la mise en œuvre d'un espace de caractéristiques comprenant de multiples expressions de caractéristiques,
dans lequel, à l'étape d), avant le paramétrage de l'image de stimulus, l'une des expressions de caractéristiques est sélectionnée dans l'espace de caractéristiques et est mise en œuvre dans l'image de stimulus,
et dans lequel, à l'étape h), avant l'ajustement du premier paramétrage, l'une des expressions caractéristiques est à nouveau sélectionnée dans l'espace de caractéristiques et mise en œuvre dans l'image de stimulus.

7. Procédé selon l'une des revendications précédentes, dans lequel le réseau neuronal est un réseau neuronal convolutif.

8. Procédé selon l'une des revendications précédentes, dans lequel les étapes b), c), d), e), g), h), j), k), l), m) et/ou n) sont exécutées par un logiciel de commande, dans lequel le logiciel de commande utilise de préférence un algorithme psychophysique ou est un algorithme psychophysique.

9. Procédé selon la revendication 8, dans lequel l'algorithme psychophysique procède selon un procédé adaptatif par étapes.

10. Procédé de production d'un moyen optique optimisé par un procédé selon l'une des revendications précédentes, comprenant
la détermination de données de traitement de surface selon le second paramétrage du moyen optique optimisé sélectionné à l'étape n),
l'envoi des données de traitement de surface à un automate de traitement mécanique de surfaces optiques en vue du traitement mécanique des surfaces optiques d'ébauches conformément aux données de traitement de surface.

11. Procédé selon la revendication 10, dans lequel le traitement mécanique des surfaces optiques comprend le meulage et/ou le polissage, les données de traitement de surface comprennent des données de meulage et/ou des données de polissage, et l'automate de traitement mécanique de surfaces optiques comprend un automate de meulage et/ou de polissage.

12. Dispositif de production de moyens optiques optimisés, comprenant
un automate de traitement mécanique de surfaces optiques, un dispositif de détermination de données de traitement de surface conformément au second paramétrage du moyen optique optimisé sélectionné à l'étape n),
un dispositif d'évaluation pour la mise en œuvre d'un procédé selon l'une des revendications 1 à 9, comprenant des interfaces pour la lecture en entrée de jeux de données d'apprentissage et pour la fourniture en entrée et/ou la sélection d'images de stimulus ainsi que de données de paramétrage destinées aux premier et second paramétrages.

13. Dispositif selon la revendication 12, comprenant en outre un dispositif de test permettant de contrôler les moyens optiques optimisés traités.

14. Programme informatique comportant un code de programme exécutable sur un processeur pour mettre en œuvre les étapes de procédé d'un procédé selon l'une des revendications 1 à 9.

15. Support de stockage non transitoire sur lequel est stocké un programme informatique selon la revendication 14.
